# EUROPEAN PATENT SPECIFICATION

(11) **EP 2 547 809 B1**
(45) Date of publication and mention of the grant of the patent: **28.10.2015**
(21) Application number: 11755585.4
(22) Date of filing: 15.03.2011
(51) Int. Cl.: C40B 40/06, C07H 21/00, C12Q 1/68, C40B 30/04, C40B 50/00

(54) **METHODS, PROBE SETS, AND KITS FOR DETECTION OF DELETION OF TUMOR SUPPRESSOR GENES BY FLUORESCENCE IN SITU HYBRIDIZATION**
VERFAHREN, SONDENSETS UND KITS ZUR ERKENNUNG DER ZERSTÖRUNG VON TUMORUNTERDRÜCKERGENEN DURCH IN-SITU-FLUORESZENZ-HYBRIDISIERUNG
MÉTHODES, ENSEMBLES DE SONDES ET KITS DE DÉTECTION DE DÉLÉTION DE GÈNES SUPPRESSEURS DE TUMEUR PAR HYBRIDATION IN SITU EN FLUORESCENCE

(30) Priority: 15.03.2010 US 313916 P; 15.03.2010 CA 2696545
(43) Date of publication of application: 23.01.2013
(73) Proprietor: Queen's University At Kingston, Kingston, Ontario K7L 3N6 (CA); Kingston General Hospital, Kingston, Ontario K7L 2V4 (CA)
(72) Inventor: SQUIRE, Jeremy, A., Kingston Ontario K7L 4N9 (CA); YOSHIMOTO, Maisa, Kingston Ontario K7K 7K6 (CA)
(74) Representative: Finnegan Europe LLP
(86) International application number: PCT/CA2011/000268
(87) International publication number: WO 2011/113138

(56) References cited:
- KANISHKA SIRCAR ET AL: "PTEN genomic deletion is associated with p-Akt and AR signalling in poorer outcome, hormone refractory prostate cancer", THE JOURNAL OF PATHOLOGY, vol. 218, no. 4, 1 August 2009 (2009-08-01), pages 505-513, XP055090284, ISSN: 0022-3417, DOI: 10.1002/path.2559
- M YOSHIMOTO ET AL: "FISH analysis of 107 prostate cancers shows that PTEN genomic deletion is associated with poor clinical outcome", BRITISH JOURNAL OF CANCER, vol. 97, no. 5, 28 August 2007 (2007-08-28), pages 678-685, XP055090286, ISSN: 0007-0920, DOI: 10.1038/sj.bjc.6603924
- OSOEGAWA K ET AL: "A bacterial artificial chromosome library for sequencing the complete human genome", GENOME RESEARCH, COLD SPRING HARBOR LABORATORY PRESS, WOODBURY, NY, US, vol. 11, no. 3, 1 March 2001 (2001-03-01), pages 483-496, XP002310986, ISSN: 1088-9051, DOI: 10.1101/GR.169601
- YOSHIMOTO ET AL.: 'Three-color FISH analysis of TMPRSS2/ERG fusions in prostate cancer indicates that genomic microdeletion of chromosome 21 is associated with rearrangement.' NEOPLASIA vol. 8, no. 6, June 2006, ISSN 1522-8002 pages 465 - 469
- YOSHIMOTO ET AL.: 'FISH analysis of 107 prostate cancers shows that PTEN genomic deletion is associated with poor clinical outcome.' BRITISH JOURNAL OF CANCER. vol. 97, no. 5, 28 August 2007, ISSN 0007-0920 pages 678 - 685
- PERNER ET AL.: 'TMPRSS2:ERG fusion-associated deletions provide insight into the heterogeneity of prostate cancer.' CANCER RESEARCH. vol. 66, no. 17, 01 September 2006, pages 8337 - 8341, XP055034511

## Description

This invention concerns methods, probe sets, and kits for use in assays for detecting deletions of tumor suppressor genes. Also described herein are methods for preparing such probe sets and optimizing such assays. Methods described herein can be used for detecting chromosomal events such as deletions, amplifications, translocations and other chromosomal events in samples such as blood or solid tumors, as in formalin-fixed paraffin embedded (FFPE) thin sections, fine-needle aspiration (FNA) biopsies, smears, etc. The chromosomal events may involve genes such as tumor suppressors, associated oncogenes and/or proto-oncogenes.

Knowledge of whether cancerous or precancerous cells are deleted for a tumor suppressor gene is generally useful for improving diagnostic and/or prognostic accuracy or choice of treatment. It is common for samples of suspected cancerous or precancerous cells, such as from suspected cancerous or precancerous growths including tumors, neoplasms, hyperplasias, etc., to be provided for laboratory analysis, for example, as fixed, preserved samples, such as formalin fixed paraffin embedded (FFPE) samples. Notably, the preservation of such samples also allows retrospective studies to be performed, which can provide information useful for making future prognoses and treatment choices. Deletion of a tumor suppressor gene can be assayed in the FFPE samples by fluorescence in situ hybridization (FISH), in which the sample is contacted with a probe for the gene of interest and, generally, at least one control probe and/or nuclear counterstain. Examples of tumor suppressor genes include PTEN, p53, p16 (also known as CDKN2A), RB1, DCC, BRCA1, BRCA2, and APC. The probes can be visualized fluorescently and the resulting FISH signals are analyzed to determine whether a deletion is present.

When samples from solid growths are provided as fixed and preserved samples, they are generally provided as sections of several microns in thickness. Therefore, the part of the nucleus that contains the tumor suppressor gene may be ablated during the sectioning of the sample. This phenomenon often results in nuclear truncation artifacts, causing cells that did not actually harbor a deletion of a tumor suppressor gene to appear as though at least one copy of the tumor suppressor gene is missing. Truncation artifacts can occur in interphase cells, in which the chromosomes are decondensed and distributed within the three dimensional volume of the spherical shaped nuclei that constitute routine pathology tissue sections. In metaphase cells, the ~46 chromosomes are present as flat, rodshaped, linear structures, which can generally be deposited on the surface of microscope slides without truncation. Indeed, in clinical samples, including samples from suspected cancerous or precancerous solid growths, such as solid tumors, the majority of the cells generally are in interphase. Representative samples of metaphase cells can be difficult or impossible to obtain from samples used for retrospective analyses, or from samples taken from a patient, fixed, preserved, and sent to an off-site laboratory for analysis.

Nuclear truncation artifacts thus negatively impact the performance of FISH-based tumor suppressor deletion assays. In order to guard against the possibility of false positives (i.e., an incorrect conclusion that a tumor suppressor deletion has occurred), a minimum threshold for apparent deletion frequency can be set, wherein results are considered negative or at most inconclusive for deletion when the apparent deletion frequency does not exceed the threshold. The threshold can be based on an estimate of the artifactual deletion frequency made using control cells; in order for an observed level of deletion to be considered significant, it may need to exceed the artifactual deletion frequency by a quantity such as three standard deviations of the artifactual deletion frequency. Use of such minimum thresholds can result in a tradeoff of sensitivity, however, in that it can be difficult or impossible to call deletions when, for example, the number of cells in the sample harboring the deletion is low, or when the cells in the sample are genetically heterogeneous.

The phosphatase and tensin homolog (PTEN) is a protein which in humans is encoded by the *PTEN* gene. Inactivation of this tumor suppressor gene has been observed in many types of cancers (Cristofano AD et al., PTEN is essential for embryonic development and tumour suppression, Nature Genetics 1998; 19:348-355) and in the inherited developmental defect known as Cowden syndrome (Marsh DJ et al., Germline PTEN mutations in Cowden syndrome-like families, J. Med. Genet. 1998; 35:881-885). In recent years, it has become apparent that genomic deletions of 368 kb or more around the *PTEN* locus are common in prostate cancer, based on the use of a 368 kb probe that hybridizes to *PTEN* (Yoshimoto et al., Cancer Genetics and Cytogenetics 2006; 169:128-137), and their presence carries an unfavorable prognosis (Yoshimoto et al., British Journal of Cancer 2007; 97:678-685; Yoshimoto et al., Modern Pathology 2008; 21:1451-1460). Thus, accurate assays for deletions of tumor suppressor genes such as *PTEN* are clinically important. Sircar et al (J. Pathol. 2009; 218: 505-513) reports that *PTEN* genomic deletions are associated with activation of p-Akt and AR signalling in poorer outcome, hormone refractory prostate cancer.

Additionally, there is a need for greater ability to detect deletions in a manner that distinguishes deletions by size. Tumors of the prostate in which there is a homozygous *PTEN* deletion, in which at least one of the deletion events eliminates an area of the chromosome larger than just *PTEN* and its immediate surroundings, may be more likely to be metastasizing tumors or have an increased potential for metastasis. Thus, probe sets and methods for such deletions can be helpful in prognosis and for making decisions about treatment, as well as in further research into the progression and classification of prostate cancers.

Accordingly, there is a need for methods, probe sets, and kits for use in assays for detecting deletions of tumor suppressor genes, such as, e.g., *PTEN,* that reduce the incidence of nuclear truncation artifacts, and methods for preparing such probe sets and optimizing such assays.

The present invention provides a method of conducting a fluorescence in situ hybridization-based assay for deletion of a tumor suppressor gene comprising:
(a) performing fluorescence in situ hybridization (FISH) with a probe set on a cellular sample comprising a plurality of cells,
   wherein the probe set comprises at least one first flanking probe that hybridizes to a position centromeric to the tumor suppressor gene, at least one second flanking probe that hybridizes to a position telomeric to the tumor suppressor gene, and at least one target probe that hybridizes to the tumor suppressor gene;
(b) enumerating FISH signals from the at least one first and at least one second flanking probes and the at least one target probe in the plurality of cells;
(c) providing at least one artifactual deletion frequency for (1) a deletion that affects only the target probe, (2) a deletion that affects the target probe and the centromeric flanking probe closest to the target probe, (3) a deletion that affects the target probe and the telomeric flanking probe closest to the target probe, or (4) a deletion that affects the target probe, the centromeric flanking probe closest to the target probe, and the telomeric flanking probe closest to the target probe,
   the artifactual deletion frequency being chosen from (i) an artifactual hemizygous deletion frequency and (ii) an artifactual homozygous deletion frequency;
(d) determining at least one apparent deletion frequency from the enumerated FISH signals of step (b), for the same type of deletion event as at least one artifactual deletion frequency of step (c),
   the apparent deletion frequency being chosen from (i) an apparent hemizygous deletion frequency and (ii) an apparent homozygous deletion frequency,
   wherein the at least one apparent deletion frequency comprises an apparent hemizygous deletion frequency if an artifactual homozygous deletion frequency was not provided in step (c), and wherein the at least one apparent deletion frequency comprises an apparent homozygous deletion frequency if an artifactual hemizygous deletion frequency was not provided in step (c); and
(e) determining whether the sample comprises cells with a hemizygous deletion of the tumor suppressor gene based on whether the apparent hemizygous deletion frequency is significantly greater than the artifactual hemizygous deletion frequency, or determining whether the sample comprises cells with a homozygous deletion of the tumor suppressor gene based on whether the apparent homozygous deletion frequency is significantly greater than the artifactual homozygous deletion frequency.

The invention also provides a probe set comprising at least one probe that hybridizes to *PTEN,* at least one probe that hybridizes to *FAS* or *SUFU,* and at least one probe that hybridizes to *WAPAL,* wherein the *WAPAL*-hybridizing probe is derived from RP11-661D10.

The methods and compositions of this invention are based in part on the discovery that the performance (specificity, sensitivity, and/or statistical significance) of FISH-based tumor suppressor deletion assays can be optimized by providing probe sets that can reduce sources of error such as truncation artifacts. The probe sets comprise at least a first flanking probe, a target probe, and a second flanking probe, which hybridize at positions centromeric to, at or adjacent to, and telomeric to the tumor suppressor gene, respectively. The tumor suppressor gene can be prone to deletion in certain types of cancerous or precancerous cells; for example, *PTEN* is prone to deletion at least in neoplasms of the prostate. The distance between the hybridization site of the first or second flanking probe and the target probe can be short on a cytogenetic length scale, for example, a distance ranging from 500 kb to 10 or 20 Mb.

In some embodiments, the hybridization sites of the first and second flanking probes are separated from the hybridization site of the target probe by first and, optionally, second boundary zones, respectively (e.g., genomic architecture features close to these sites such as segmental duplications and copy number variations; discussed further below). The hybridization sites of the first and second flanking probes can be within or adjacent, on a cytogenetic length scale, to the first and second (if present) boundary zones, respectively.

The probe sets can optimize the performance of FISH-based assays for genomic aberrations because of the position of their hybridization sites. Positioning flanking probes near the hybridization site of the target probe and/or adjacent to a boundary zone, but distal to the assay target relative to the boundary zone, can serve to minimize inconclusive results and/or artifactual observations, including false positives resulting from nuclear truncation.

Accordingly, this disclosure describes methods of conducting FISH-based assays, methods of preparing probes for FISH-based tumor suppressor deletion assays, and methods of measuring the effect of nuclear truncation in a FISH-based assay for tumor suppressor deletion. This disclosure also describes probe sets and compositions and kits comprising probes useful for FISH-based tumor suppressor deletion assays.

A method of preparing a probe set for a FISH-based tumor suppressor deletion assay, may comprise:
(a) identifying at least one boundary zone on a chromosome, said chromosome comprising a tumor suppressor gene, wherein the at least one boundary zone comprises a first boundary zone centromeric to the tumor suppressor gene;
(b) providing at least a first flanking probe that hybridizes to a nucleic acid sequence within the first boundary zone or to a nucleic acid sequence distal to the tumor suppressor gene relative to the first boundary zone;
(c) providing at least a second flanking probe that hybridizes to a nucleic acid sequence telomeric to the tumor suppressor gene; and
(d) providing at least one target probe that hybridizes to a nucleic acid sequence in the tumor suppressor gene between the boundary zones.

A method of conducting a FISH-based assay for deletion of a tumor suppressor gene may comprise:
(a) performing FISH with a probe set on a cellular sample comprising a plurality of cells,
   wherein the probe set comprises at least a first flanking probe that hybridizes to a position centromeric to the tumor suppressor gene, at least a second flanking probe that hybridizes to a position telomeric to the tumor suppressor gene, and at least one target probe that hybridizes to the tumor suppressor gene;
(b) enumerating FISH signals from the at least first and at least second flanking probes and the at least one target probe in the plurality of cells;
(c) providing at least one artifactual deletion frequency chosen from (i) an artifactual hemizygous deletion frequency and (ii) an artifactual homozygous deletion frequency;
(d) determining at least one apparent deletion frequency chosen from (i) an apparent hemizygous deletion frequency and (ii) an apparent homozygous deletion frequency from the enumerated FISH signals of step (b), wherein the at least one apparent deletion frequency comprises an apparent hemizygous deletion frequency if an artifactual homozygous deletion frequency was not provided in step (c), and wherein the at least one apparent deletion frequency comprises an apparent homozygous deletion frequency if an artifactual hemizygous deletion frequency was not provided in step (c); and
(e) determining whether the sample comprises cells with a hemizygous deletion of the tumor suppressor gene based on whether the apparent hemizygous deletion frequency is significantly greater than the artifactual hemizygous deletion frequency, or determining whether the sample comprises cells with a homozygous deletion of the tumor suppressor gene based on whether the apparent homozygous deletion frequency is significantly greater than the artifactual homozygous deletion frequency.

A method of conducting a FISH-based assay for distinguishably detecting small and large deletions of a tumor suppressor gene may comprise:
(a) performing FISH on a cellular sample comprising a plurality of cells with a probe set, or performing FISH on a first cellular sample comprising a plurality of cells with a first probe subset comprised by a probe set and performing FISH on a second cellular sample comprising a plurality of cells from the same individual as the first cellular sample with a second probe subset comprised by said probe set,
   wherein the probe set comprises at least one target probe that hybridizes to the tumor suppressor gene, at least a first flanking probe that hybridizes to a position centromeric to the tumor suppressor gene, at least a second flanking probe that hybridizes to a position telomeric to the tumor suppressor gene,
   and at least one of at least a third flanking probe that hybridizes to a position centromeric to the hybridization site of the first flanking probe and at least a fourth flanking probe that hybridizes to a position telomeric to the hybridization site of the second flanking probe;
(b) enumerating FISH signals from the at least one target probe and the at least first, at least second, and at least one of the at least third and at least fourth flanking probes in the plurality or pluralities of cells;
(c) providing at least one first artifactual deletion frequency for deletions of the tumor suppressor gene with endpoints between the at least first and at least second flanking probes;
(d) providing at least one second artifactual deletion frequency for deletions of the tumor suppressor gene wherein at least one of the endpoints is not between the at least first and at least second flanking probes;
(e) determining, from the enumerated FISH signals of step (b), at least one first apparent deletion frequency for deletions of the tumor suppressor gene with endpoints between the at least first and at least second flanking probes;
(f) determining, from the enumerated FISH signals of step (b), at least one second apparent deletion frequency for deletions of the tumor suppressor gene wherein at least one of the endpoints is not between the at least first and at least second flanking probes; and
(g) determining whether the sample comprises cells with a small deletion of the tumor suppressor gene based on whether the at least one first apparent deletion frequency is significantly greater than the at least one first artifactual deletion frequency, and determining whether the sample comprises cells with a large deletion of the tumor suppressor gene based on whether the at least one second apparent deletion frequency is significantly greater than the at least one second artifactual homozygous deletion frequency.

A method of optimizing a FISH-based assay for deletion of a tumor suppressor gene, may comprise:
(a) providing a plurality of candidate probe sets, wherein each candidate probe set comprises at least a first flanking probe that hybridizes to a position centromeric to the tumor suppressor gene, at least a second flanking probe that hybridizes to a position telomeric to the tumor suppressor gene, and at least one target probe that hybridizes to the tumor suppressor gene;
(b) for each candidate probe set,
   (i) performing FISH with the candidate probe set on at least one cellular sample comprising a plurality of cells comprising a euploid number of intact copies of the tumor suppressor gene;
   (ii) enumerating FISH signals from the at least first and at least second flanking probes and the at least one target probe of the candidate probe set in the plurality of cells of the at least one sample; and
   (iii) determining an artifactual deletion frequency from the enumerated FISH signals of step (ii); and
(c) selecting a probe set from the candidate probe sets for use in the optimized FISH-based assay for deletion of a tumor suppressor gene, wherein the selected probe set was determined to have a favorable artifactual deletion frequency in step (iii).

A method of conducting a FISH-based assay for a deletion in bands 21q22.13-21q22.3 of chromosome 21 may comprise:
(a) performing FISH with a probe set on a cellular sample comprising a plurality of cells,
   wherein the probe set comprises:
   at least one target probe that hybridizes to at least one target gene located between *TMPRSS2* and *ERG* or alternatively, located within the region encompassed by the *TMPRSS2* and *ERG* genes,
   at least a first flanking probe that hybridizes to a position centromeric to the at least one target gene,
   and at least a second flanking probe that hybridizes to a position telomeric to the at least one target gene;
(b) enumerating FISH signals from the at least first and at least second flanking probes and the at least one target probe in the plurality of cells;
(c) providing at least one artifactual deletion frequency chosen from (i) an artifactual hemizygous deletion frequency and (ii) an artifactual homozygous deletion frequency;
(d) determining at least one apparent deletion frequency chosen from (i) an apparent hemizygous deletion frequency and (ii) an apparent homozygous deletion frequency from the enumerated FISH signals of step (b), wherein the at least one apparent deletion frequency comprises an apparent hemizygous deletion frequency if an artifactual homozygous deletion frequency was not provided in step (c), and wherein the at least one apparent deletion frequency comprises an apparent homozygous deletion frequency if an artifactual hemizygous deletion frequency was not provided in step (c); and
(e) determining whether the sample comprises cells with a hemizygous deletion of at least one of the target genes based on whether the apparent hemizygous deletion frequency is significantly greater than the artifactual hemizygous deletion frequency, or determining whether the sample comprises cells with a homozygous deletion of at least one of the target genes based on whether the apparent homozygous deletion frequency is significantly greater than the artifactual homozygous deletion frequency.

A method of conducting a FISH-based assay for distinguishably detecting small and large deletions in bands 21q22.13-21q22.3of chromosome 21 may comprise:
(a) performing FISH on a cellular sample comprising a plurality of cells with a probe set, or performing FISH on a first cellular sample comprising a plurality of cells with a first probe subset comprised by a probe set and performing FISH on a second cellular sample comprising a plurality of cells from the same individual as the first cellular sample with a second probe subset comprised by said probe set,
   wherein the probe set comprises:
   at least two target probes that hybridize to at least two target genes located between TMPRSS2 and ERG,
   at least a first flanking probe that hybridizes to a position centromeric to the at least two target genes,
   at least a second flanking probe that hybridizes to a position telomeric to the at least two target genes;
   and, optionally, at least one of at least a third flanking probe that hybridizes to a position centromeric to the hybridization site of the first flanking probe and at least a fourth flanking probe that hybridizes to a position telomeric to the hybridization site of the second flanking probe;
(b) enumerating FISH signals from the at least two target probes and the at least first, at least second, and, if present, at least one of the at least third and at least fourth flanking probes in the plurality or pluralities of cells;
(c) providing at least one first artifactual deletion frequency for deletions of at least one of the target genes with endpoints between the at least first and at least second flanking probes, one of the endpoints being between two target genes;
(d) providing at least one second artifactual deletion frequency for deletions of at least one of the target genes wherein (i) neither endpoint is between two target genes, or (ii) if at least one of the at least third and at least fourth flanking probes was used, at least one of the endpoints is hot between the at least first and at least second flanking probes;
(e) determining, from the enumerated FISH signals of step (b), at least one first apparent deletion frequency for deletions of at least one of the target genes with endpoints between the at least first and at least second flanking probes, one of the endpoints being between two target genes;
(f) determining, from the enumerated FISH signals of step (b), at least one second apparent deletion frequency for deletions of at least one of the target genes wherein (i) neither endpoint is between two target genes, or (ii) if at least one of the at least third and at least fourth flanking probes was used, at least one of the endpoints is not between the at least first and at least second flanking probes; and
(g) determining whether the cellular sample comprises, or the first and second cellular samples comprise, cells with a small deletion of at least one of the target genes based on whether the at least one first apparent deletion frequency is significantly greater than the at least one first artifactual deletion frequency, and determining whether the cellular sample comprises, or the first and second cellular samples comprise, cells with a large deletion of at least one of the target genes based on whether the at least one second apparent deletion frequency is significantly greater than the at least one second artifactual homozygous deletion frequency.

Also described herein is a probe set comprising at least one probe that hybridizes to PTEN, at least one probe that hybridizes to FAS or SUFU, and at least one probe that hybridizes to TSPAN15.

Also described herein is a probe set comprising at least one probe that hybridizes to at least one target gene located between TMPRSS2 and ERG, at least one probe that hybridizes to DYRK1A or ERG, and at least one probe that hybridizes to TMPRSS2 or U2AF1.

It is to be understood that both the foregoing general description and the following description of embodiments are exemplary and explanatory only and are not restrictive of the invention, as claimed.

### DESCRIPTION OF EMBODIMENTS

### A. Overview

Described herein are methods of conducting FISH-based assays; methods of preparing probes for FISH-based tumor suppressor deletion assays; methods of optimizing a FISH-based assay for deletion of a tumor suppressor gene; and probe sets, compositions, and kits comprising probes useful for FISH-based tumor suppressor deletion assays.

The tumor suppressor may be chosen from PTEN, p53, p16 (also known as CDKN2A), RB1, DCC, BRCA1, BRCA2, and APC. These tumor suppressors are discussed in, for example, Chang H et al., Multiple myeloma involving central nervous system: high frequency of chromosome 17p13.1 (p53) deletions, British Journal of Haematology 2004; 127:280-284; Kohno T and Yokota J, Molecular processes of chromosome 9p21 deletions causing inactivation of the p16 tumor suppressor gene in human cancer: Deduction from structural analysis of breakpoints for deletions, DNA Repair 2006; 5:1273-1281; Friend SH et al., A human DNA segment with properties of the gene that predisposes to retinoblastoma and osteosarcoma, Nature 1986; 323:643-6; Popat S and Houlston RS, A systematic review and meta-analysis of the relationship between chromosome 18q genotype, DCC status and colorectal cancer prognosis, European Journal of Cancer 2005; 41:2060-2070; Becker K et al., Deletions of BRCA1/2 and p53 R248W gain-of-function mutation suggest impaired homologous recombination repair in fragile histidine triad negative sebaceous gland carcinomas, British Journal of Dermatology 2008; 159:1282-1289; and Castellsagué E et al., Detection of APC gene deletions using quantitative multiplex PCR of short fluorescent fragments, Clin. Chem. 2008; 54:1132-40.

The tumor suppressor may be chosen from a tumor suppressor located on a human chromosome at a chromosome band chosen from 10q23, 17p13, 13q14, 9q24, and 9p21. The tumor suppressor may be chosen from a tumor suppressor located on a human chromosome arm chosen from 10q, 17p, 13q, 9p, 1p, 5q, 19q, 20q, 8p, 12p, and 16q. The presence of tumor suppressors in the foregoing bands and arms is discussed, for example, in Kolomietz E et al., Quantitative PCR identifies a minimal deleted region of 120kb extending from the Philadelphia chromosome ABL translocation breakpoint in chronic myeloid leukemia with poor outcome, Leukemia 2003; 17: 1313-1323, 2003; Haase D, Cytogenetic features in myelodysplastic syndromes, Ann. Hematol. 2008; 87:515-526; Reifenberger J et al., Molecular genetic analysis of oligodendroglial tumors shows preferential allelic deletions on 19q and 1p, Am. J. Pathol. 1994; 145:1175-1190; Chang B et al., Integration of Somatic Deletion Analysis of Prostate Cancers and Germline Linkage Analysis of Prostate Cancer Families Reveals Two Small Consensus Regions for Prostate Cancer Genes at 8p, Cancer Research 2007; 67:4098-4103; and Reiner M et al., Microarray comparative genomic hybridization analysis of tubular breast carcinoma shows recurrent loss of the CDH13locus on 16q, Human Pathology 2008; 39:1621-1629.

Human chromosome arms and bands are as defined in ISCN 2009. An International System for Human Cytogenetics Nomenclature, Editors: Shaffer LG, Slovak ML, Campbell LJ., 2009; Chapter 2, S. Karger Publishers Inc.

The definitions below are definitions of terms and concepts useful in understanding embodiments of the invention and are followed by a discussion of the embodiments in detail and examples of those embodiments. While the discussion of the embodiments is organized into sections and subsections, it is to be understood that many of the aspects discussed in one subsection have relevance to embodiments in other sections; for example, a discussion of types of probes is relevant to methods that use the probes.

### B. Definitions

As used herein, a "boundary zone" is a region of a chromosome in which endpoints of deletions occur at increased frequency. The location of boundary zones near a tumor suppressor gene on a chromosome can be determined by measuring endpoints of deletions for a number of samples, and identifying a region in which many of the endpoints are clustered. Deletion endpoints in a population of samples can be measured by techniques such as FISH or comparative genomic hybridization (CGH) using arrays; this is discussed further below in section C.1. In some embodiments, the sizes of boundary zones are greater than or equal to 50 kb, 100 kb, 200 kb, 300 kb, 400 kb, 500 kb, or 1 Mb, and/or less than or equal to 1.5 Mb, 2 Mb, 3 Mb, 4 Mb, or 5 Mb. Boundary zones include fragile regions identified by CGH.

For a given tumor suppressor gene, zone size, and condition of interest (e.g., prostate cancer), the chromosome comprising the tumor suppressor gene may comprise, in some embodiments, "primary boundary zones." A primary boundary zone is a region centromeric or telomeric to a tumor suppressor gene that comprises deletion endpoints at the greatest frequency. There can be two primary boundary zones, one to each side of the tumor suppressor gene.

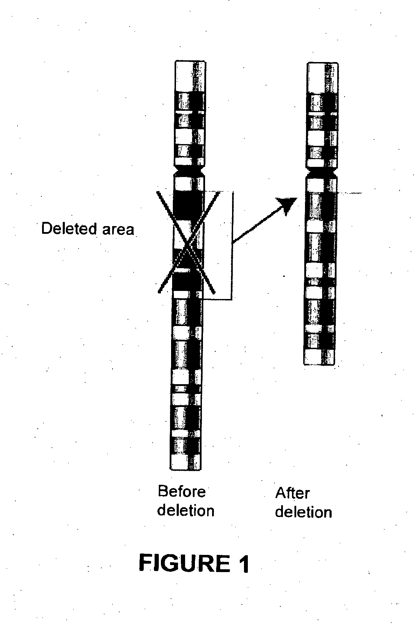

The term "deletion" is used herein in a cytogenetic sense to describe a structural chromosome aberration in which a part of a chromosome arm is missing. Thus, deletion is the loss of chromosomal material and the gene(s) that map to the particular region of the genome that is missing. Broadly speaking, FISH probes, such as probes prepared by labeling DNA from bacterial artificial chromosomes (BACs), are hybridized to a cellular sample, and the presence or absence of FISH signal is used to determine if a deletion is present. The simplest type of deletion encountered clinically is an interstitial deletion (see Figure 1). Some deletions are considered to be microdeletions, which are below the resolution of classical cytogenetic analyses which is ∼ 5Mb DNA. Such submicroscopic interstitial losses of DNA occur from the interior of a chromosome and are likely to occur within one cytoband. They may be as small as a few hundred kb of DNA and are typically detected by FISH methods.

As used herein, a "probe set" is a group of DNA sequences differentially labeled or able to be differentially labeled for use in a multicolor FISH assay. The probes of a probe set can be prepared by various procedures known to those of skill in the art, including, without limitation, by isolation of BAC or cosmid DNA from host strains; amplification (e.g., polymerase chain reaction or rolling circle amplification) of genomic or cloned DNA; or artificial synthesis, e.g., of a set of oligonucleotides that cover a sufficiently large region in the target genome (e.g., at least 50 kb, or at least 100 kb) to generate signals visible by fluorescence microscopy. It should be noted that a probe for a given region may be derived from a plurality of bacterial artificial chromosomes (BACs), cosmids, amplification products, oligonucleotides, etc. In some cases, the probe may be designed not to contain repeated sequences that would also hybridize to other regions of the genome. It is to be understood that a probe set "comprising" probes "derived from" specified BACs is open with respect to additional BACs being used, either as contributing to explicitly recited probes or to provide or contribute to additional probes. In contrast, a probe set "consisting of" probes "derived from" specified BACs is closed with respect to additional BACs being used. A probe that "hybridizes to" a specified gene has a binding site that overlaps the specified gene at least in part. The probe may or may not also hybridize to part of the neighboring genes. The term "gene" is generally used as referring to an area of genomic DNA bounded by chromosomal coordinates such as are provided for a given gene in the GeneCards® database, v3.05, Feb. 13, 2011 (See, e.g., http://www.genecards.org).

As used herein, a "FISH signal" is a spot of fluorescence observed in a FISH assay resulting from hybridization of a probe to its target. FISH signals often are pointlike but may not always appear totally compacted, depending on the condensation state of the chromatin in the cell under observation.

When a cellular sample comprising a plurality of cells with a given characteristic is discussed, it should be noted that it is not necessarily the case that all the cells in the cellular sample have the given characteristic.

As used herein, "artifactual deletion frequency" refers to the frequency in a deletion assay with which a lack of a FISH signal is observed due to sources of error, such as truncation of nuclei during sample preparation or any other cause unrelated to the genotype of the cells in the sample. Artifactual deletion frequencies can be determined using negative control samples which do not contain deletions of the gene targeted in the FISH assay.

As used herein, "apparent deletion frequency" refers to the frequency in a deletion assay with which a lack of a FISH signal is observed; it is thus the combination of the artifactual deletion frequency (which is generally unknown for a test sample, but can be estimated from a control sample as discussed above) with the frequency of actual deletions in the sample. An apparent deletion frequency can refer to the frequency of the absence of a specific set of one or more FISH signals, to the overall frequency with which one FISH signal from the target probe is missing, to the overall frequency with which two FISH signals from the target probe is missing, or to the overall frequency with which at least one FISH signal from the target probe is missing.

As used herein, "euploid number" refers to the number of copies of a gene that would be normal for a cell of a given type. A typical value of the euploid number is 2 for somatic chromosomal loci in somatic cells, except after replication and prior to division when the euploid number is 4. There are other situations in which the euploid number differs, which are known to those of skill in the art; for example, the euploid number is reduced by half in germline cells that have undergone a reductive meiotic division, and in male cells for X chromosome loci. The euploid number for Y chromosome loci is 0 in female cells and 1 (or 2, post-replication and pre-division) in male cells.

A "chromosome enumeration probe" refers to a probe used to determine the number of a specific chromosome in a nucleus; common types of chromosome enumeration probes include probes hybridizing at centromeric or pericentromeric loci.

"Favorable" when used with reference to artifactual deletion frequency and/or sensitivity values given by a candidate probe set means that the values are at least better than the average values of the candidate probe sets tested. It is of course also possible to select a candidate probe set by applying more stringent criteria, such as that the artifactual deletion frequency and/or sensitivity values be in at least the 60^{th}, 70^{th}, 80^{th}, 90^{th}, or 95^{th} percentile, it being understood that higher percentiles indicate better performance (i.e., higher sensitivity or lower artifactual deletion frequency).

A first locus is "centromeric" to a second locus if the first locus is closer to the centromere than the second locus.

A first locus is "telomeric" to a second locus if the first locus is closer than the second locus to the telomere of the arm where the first locus occurs.

When a first locus is "distal" to a second locus relative to a third, such as a hybridization site being distal to a tumor suppressor relative to a boundary zone, it means that the first locus is farther from the second locus than it is from the third locus; thus, if a hybridization site is distal to a tumor suppressor relative to a boundary zone that is centromeric to the tumor suppressor, the hybridization site is necessarily centromeric to the tumor suppressor. Likewise, if a hybridization site is distal to a tumor suppressor relative to a boundary zone that is telomeric to the tumor suppressor, the hybridization site is necessarily telomeric to the tumor suppressor.

### C. Methods of preparing probes

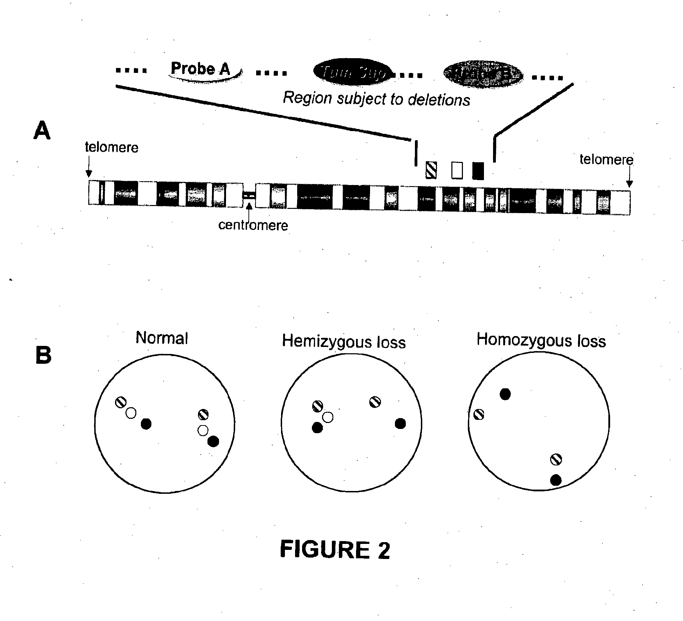

The dsclosure herein relates, in part, to preparing probes for three or more color FISH assays for deletion detection. The probes can be designed to have hybridization sites within and at or near the ends of a genomic interval of, e.g., between 0.5-10 megabases (mb) of DNA that is frequently deleted in human cancers. A target probe is prepared that hybridizes to the tumor suppressor gene, and flanking probes are provided that have hybridization sites telomeric and centromeric to the tumor suppressor gene (Figure 2A).

The flanking probes prepared according to methods described herein can have hybridization sites within identified boundary zones and/or distal to a tumor suppressor gene relative to a boundary zone. A flanking probe may have a hybridization site distal to a tumor suppressor gene relative to a boundary zone, wherein the center of the hybridization site is near the proximal edge of the boundary zone, for example, less than or equal to 2 Mb, 1.5 Mb, 1 Mb, 500 kb, 200 kb, or 100 kb from the proximal edge of the boundary zone.

### 1. Boundary zone identification

Boundary zones can be identified using comparative genomic hybridization data derived from public domain datasets such as are available at [http://www.broadinstitute.org/tumorscape/pages/portalHome.jsf], described in Beroukhim R et al., The landscape of somatic copy-number alteration across human cancers, Nature 2010; 463:899-905; see also Liu W et al., Copy number analysis indicates monoclonal origin of lethal metastatic prostate cancer, Nat Med 2009; 15:559-65, and Ferreira BI et al., Array CGH and gene-expression profiling reveals distinct genomic instability patterns associated with DNA repair and cell-cycle checkpoint pathways in Ewing's sarcoma, Oncogene 2008; 27:2084-2090. Typically these datasets have been obtained by performing comparative hybridization experiments with labeled DNA from a plurality of cancerous samples, e.g., tumor samples, likely to comprise a deletion of the tumor suppressor of interest to high-resolution genomic microarrays along with distinguishably labeled reference DNA from normal cells that do not comprise a deletion. Once the genomic DNA from the plurality of samples has been determined to comprise a subset with a frequent deletion of the tumor suppressor of interest, then a boundary zone can be identified as a region in which there is a cluster of deletion breakpoints identified as distinct copy number transitions in individual samples. This can be identified from the average abundance by position in the population of cancerous samples as a marked decline in copy number with position as one moves closer to the tumor suppressor of interest. For example, the average copy number relative to the reference may decline by an amount such as 15%, 20%, or 25% over a length such as 500 kb, 750 kb, 1 Mb, 1.5 Mb, or 2 Mb.

When analysis of a large series of array comparative genomic hybridizations (e.g., 50, 75, 100, or more) is performed to assay the plurality of samples individually, boundary zones can also be identified by determining where copy number transitions occur in each sample, binning these locations (e.g., in bins corresponding to stretches of DNA of a size such as 50 kb, 100 kb, 200 kb, 300 kb, 400 kb, 500 kb, 1 Mb, 1.5 Mb, or 2 Mb), and identifying bins that are significantly enriched for copy number transitions. Copy number transitions in individual samples can be identified, for example, as described in Ferreira BI et al., Array CGH and gene-expression profiling reveals distinct genomic instability patterns associated with DNA repair and cell-cycle checkpoint pathways in Ewing's sarcoma, Oncogene 2008; 27:2084-2090. Rank segmentation can be used to identify segments of gain and loss; this process is implemented, for example, in the commercially available software Nexus Copy Number version 4 or 5 (February 2010) (BioDiscovery, EI Segundo, CA), as described in the user manual.

In addition to the above mode of boundary zone determination classes of sequence microhomology called "segmental duplications" and other classes called "copy number variation" (CNVs) may be present in the vicinity of a copy number transition identified as described above. In some embodiments, when at least one of these classes or both classes of such sequences are present adjacent to, or near (e.g., within 25, 50, 75, or 100 kb of) a copy number transition, the region will be selected for FISH probe analyses as described below. Segmental duplication positions can be obtained from the Segmental Duplication Database (She X et al., Shotgun sequence assembly and recent segmental duplications within the human genome, Nature 2004; 431:927-930). CNV data can be obtained from the Sanger Institute's CNV Project (http://www.sanger.ac.uk/humgen/cnv/); see, e.g., Redon R et al., Global variation in copy number in the human genome, Nature 2006; 444:444-454, and Komura D et al., Genome-wide detection of human copy number variations using high-density DNA oligonucleotide arrays, Genome Res. 2006; 16:1575-84. CNVs and segmental duplications are also discussed in Stankiewicz P, Lupski JR, Genome architecture, rearrangements and genomic disorders, Trends Genet. 2002; 18:74-82, Beroukhim R et al., The landscape of somatic copy-number alteration across human cancers, Nature 2010; 463:899-905, and Casci T, Genome evolution: CNV evolution revisited, Nature Reviews Genetics 2008; 9:814-815.

In some methods, it is determined whether a cluster of segmental duplications is present near a copy number transition. A cluster can comprise at least three, four, five, six, or more segmental duplications within a 500 kb, 1 Mb, or 2 Mb region.

Alternatively or in addition to the above mode for boundary zone determination, boundary zones can be identified using FISH, by assaying a large plurality (e.g., 300 or more samples) likely to comprise a deletion of the tumor suppressor of interest with a series of probes with hybridization sites spaced along the normal sequence of a chromosome. Probes in the series are considered to be neighboring if there is not a probe that binds between them. A boundary zone is determined to be present between two neighboring probes if the probe farther from the tumor suppressor of interest generates FISH signals in the samples that comprise a deletion significantly more often than the probe nearer to the tumor suppressor of interest. Significance thresholds are discussed below.

### 2. Hybridization sites

A probe is considered to hybridize to a site if the probe stably forms base pairs to a degree sufficient for detection by FISH under low stringency conditions, such as 45ºC in 2X SSC with 50% formamide. Generally, lower stringency is associated with low temperature and high salt concentration (Bayani J, Squire JA, Fluorescence in situ Hybridization (FISH), Curr Protoc Cell Biol. 2004; Chapter 22: Unit 22.4).

The hybridization sites of probes prepared according to methods described herein can be positioned various ways, including one or more of the following.

### (a) Positioning relative to targets and other hybridization sites

The methods of preparing probe sets described herein typically comprise providing at least one target probe that hybridizes to a nucleic acid sequence in a tumor suppressor gene, and at least two flanking probes, one of which hybridizes to a site centromeric to the tumor suppressor gene, and one of which hybridizes telomeric to the tumor suppressor gene.

### (b) Positioning relative to first and second boundary zones

The flanking probes can be positioned relative to boundary zones. One flanking probe can hybridize to a nucleic acid sequence within the first boundary zone or to a nucleic acid sequence distal to the tumor suppressor gene relative to the first boundary zone, and another flanking probe can hybridize to a nucleic acid sequence within the second boundary zone or to a nucleic acid sequence distal to the tumor suppressor gene relative to the second boundary zone. These sequences may be distal to the tumor suppressor gene relative to the first or second boundary zone.

### (c) Positioning relative to additional boundary zones

The methods can further comprise identifying additional boundary zones (such as a third, fourth, or fifth boundary zone) and, for each additional boundary zone, providing a probe that hybridizes to a nucleic acid sequence within the additional boundary zone or to a nucleic acid sequence distal to the tumor suppressor gene relative to the additional boundary zone; the hybridization site may be near the boundary zone. When more than two boundary zones are identified, it is to be expected that deletion size is prone to vary among samples. The probe sets can be used to obtain information about the sizes of deletions, based on whether FISH signals from one or more of the flanking probes closer to the target probe are missing as well as the FISH signal from the target probe itself.

### 3. Probe size and origin

In addition to identifying boundary zones, the methods of preparing probe sets comprise providing probes. DNA for use as a probe can be obtained, for example, by isolating BAC or cosmid DNA from one or more pre-existing host strains or BAC/cosmid libraries, or constructing one or more new BACs or cosmids; amplifying (e.g., polymerase chain reaction or rolling circle amplification) genomic or cloned DNA; or artificially synthesizing DNA, e.g., a set of oligonucleotides that cover a sufficiently large region in the target genome (e.g., at least 50 kb, or at least 100 kb) to generate signals visible by fluorescent microscopy. The size of the region hybridized by a probe can be greater than or equal to 50 kb or 100 kb, and/or less than or equal to 150 kb, 200 kb, 300 kb, 400 kb, or 500 kb.

### 4. Probe functionality

At least one target probe and at least first and second flanking probes prepared according to methods described herein can be used in a FISH-based tumor suppressor deletion assay wherein the assay has a significance threshold determined as artifactual deletion frequency plus three standard deviations) below 20%, 25%, 30%, or 35% on formalin-fixed, paraffin-embedded cellular samples with a 5 µm thickness and with an average nuclear diameter less than 5 µm. Intact nuclei in FFPE sections from samples such as cancerous prostate samples may not be present as perfect spheres but tend to be compressed slightly into an elliptical shape during section preparation.

### D. Probe sets and kits

Probe sets may be prepared according to the methods described above.

Probe sets may comprise at least one probe that hybridizes to *PTEN,* at least one probe that hybridizes to FAS or *SUFU,* and at least one probe that hybridizes to *TSPAN15.* In such probe sets, the at least one probe that hybridizes to *PTEN* serves as a target probe, and the at least one probe that hybridizes to FAS or *SUFU* and the at least one probe that hybridizes to *TSPAN15* serve as flanking probes. The probe set may comprise at least one additional flanking probe that hybridizes to *BMPR1A.* The probe set may comprise at least one additional flanking probe that hybridizes to *WAPAL.*

The at least one probe that hybridizes to *PTEN* may comprise a probe derived from the BAC RP11-846G17. The probe set may comprise at least one probe that hybridizes to FAS derived from at least one of the BACs RP11-399019 and RP11-360H20. The probe set may comprise at least one probe that hybridizes to TSPAN15 derived from at least one of the BACs RP11-404C6 and RP11-6P16. The probe set may comprise at least one probe that hybridizes to *BMPR1A* derived from at least one of RP11-141 D8 and RP11-52G13. The probe set may comprise at least one probe that hybridizes to WAPAL and/or *BMPR1A* derived from at least one of RP11-351 D13, RP11-661D10, RP11-141 D8, and RP11-52G13The probe set may comprise at least one probe that hybridizes to *WAPAL* derived from at least one of RP11-141 D8 and RP11-661D10. The probe set may comprise at least one probe that hybridizes to *SUFU* derived from at least one of RP11-18I14 and RP11-2F13. These and all other BACs mentioned herein are available from the Roswell Park Cancer Institute, Buffalo, New York, and the BACPAC Resources Center at Children's Hospital and Research Center at Oakland, California. Sequence information is available, for example, from the NCBI Clone Registry (http://www.ncbi.nlm.nih.gov/ projects/genome/clone/).

Below is a table providing information about hybridization site locations on chromosome 10 (coordinates are as in version NCBI36/hg18, March 2006) for selected BACs that can be used in probe sets according to the disclosure.

| | Start | End |
|---|---|---|
| **Centromeric (10q11.21)** | | |
| RP11-89J23 | 43,432,326 | 43,596,132 |
| RP11-1044H3 | 43,572,030 | 43,801,357 |
| RP11-80C16 | 43,723,472 | 43,889,995 |
| | | |

| ***TSPAN15* (10q21.3)** | | |
|---|---|---|
| RP11-404C6 | 70,784,881 | 70,979,123 |
| RP11-6P16 | 70,824,041 | 71,006,065 |
| | | |

| ***BMPR1A*/*WAPAL* (10q23.2)** | | |
|---|---|---|
| RP11-351D13 | 87,894,018 | 88,064,908 |
| RP11-661D10 | 88,057,057 | 88,255,780 |
| RP11-141D8 | 88,280,060 | 88,465,884 |
| RP11-52G13 | 88,441,801 | 88,565,254 |
| | | |

| ***PTEN* (10q23.31)** | | |
|---|---|---|
| RP11-846G17 | 89,666,216 | 89,843,092 |
| | | |

| ***FAS* (10q23.31)** | | |
|---|---|---|
| RP11-399019 | 90,596,403 | 90,783,731 |
| RP11-360H20 | 90,679,956 | 90,860,541 |
| | | |

| ***SUFU* (10q24.32)** | | |
|---|---|---|
| RP11-18I14 | 104,104,382 | 104,271,861 |
| RP11-2F13 | 104,232,698 | 104,414,392 |

The probe set may comprise probes that hybridize to PTEN, FAS, and TSPAN15, wherein the at least one probe that hybridizes to PTEN comprises a probe derived from the BAC RP11-846G17, the at least one probe that hybridizes to FAS comprises a probe derived from the BACs RP11-399O19 and RP11-360H20, and the at least one probe that hybridizes to TSPAN15 comprises a probe derived from the BACs RP11-404C6 and RP11-6P16.

The probe set may comprise at least one centromeric or pericentromeric probe. This can be used for chromosome enumeration. Examples of pericentromeric probes include probes derived from the BACs RP11-89J23, RP11-1044H3, and/or RP11-80C16.

The probe set may consist of probes derived from RP11-846G17, at least one of RP11-399O19 and RP11-360H20, and at least one of RP11-404C6 and RP11-6P16.

The probe set may consist of probes derived from RP11-846G17, at least one of RP11-399O19 and RP11-360H20, and at least one of RP11-141 D8 and RP11-52G13.

Generally, a probe can be derived from a BAC by performing a labeling reaction such as nick translation to generate the probe. However, the derivation may be simply to grow and isolate the BAC, in the case where the probe is provided in unlabelled form; in other words, derivatives of BACs include the BACs themselves.

The invention provides a probe set comprising at least one probe that hybridizes to PTEN, at least one probe that hybridizes to FAS or SUFU, and at least one probe that hybridizes to WAPAL, wherein the WAPAL-hybridizing probe is derived from RP11-661D10.

### 1. Probe labeling

The probe sets described herein can be labelled. It is also possible to provide the probe set in unlabelled form, but in a manner (e.g., as separate solutions or lyophilizates) that allows distinguishable labeling of the probes prior to hybridization.

The labeling can be direct fluorescent labeling. This can be achieved by well-known methods such as nick translation or random priming in the presence of directly labelled nucleotide analog. Indirect labeling is also possible, in which a nucleotide analog is provided in a labeling reaction such as a nick translation or random priming reaction that does not itself bear a fluorophore but bears a moiety that allows covalent or noncovalent attachment of a fluorophore at a later time. See, e.g., Bayani J, Squire JA. Fluorescence in situ Hybridization (FISH), Curr Protoc Cell Biol. 2004; Chapter 22: Unit 22.4. For example, nucleotide analogs bearing an aminoallyl moiety can be covalently conjugated with a fluorophore, and nucleotide analogs bearing a biotin or digoxygenin moiety can be bound noncovalently by a binding partner such as avidin or anti-digoxygenin, respectively, with the binding partner being fluorescently tagged. It is also possible to amplify signal by providing multiple layers of binding partner, such as a second binding partner (e.g., antibody) that recognizes the first binding partner and is also labeled, and possibly then a third binding partner that recognizes the second, and so on.

Examples of fluorophores that can be used as labels with the probes and methods described herein are: SpectrumGreen, SpectrumOrange, SpectrumRed, and SpectrumAqua (all from Abbott Molecular, Inter Medico, Markham, ON, Canada); 7-amino-4-methylcoumarin-3-acetic acid (AMCA); Texas Red(TM) (Molecular Probes, Inc., Eugene, Oreg.); 5-(and-6)-carboxy-X-rhodamine; lissamine rhodamine B; 5-(and-6)-carboxyfluorescein; fluorescein-5-isothiocyanate (FITC); 7-diethylaminocoumarin-3-carboxylic acid; tetramethylrhodamine-5-(and-6)-isothiocyanate; 5-(and-6)-carboxytetramethylrhodamine; 7-hydroxycoumarin-3-carboxylic acid; 6-[fluorescein 5-(and-6)-carboxamido]hexanoic acid; N-(4,4-difluoro-5,7-dimethyl-4-bora-3a,4a diaza-3-indacenepropionic acid; eosin-5-isothiocyanate; erythrosine-5-isothiocyanate; 5-(and-6)-carboxyrhodamine 6G; and Cascade(TM) blue acetylazide (Molecular Probes, Inc., Eugene, Oreg.). In the LAVysion probe set, fluorophores of different colors are used such that each chromosomal probe in the set can be distinctly visualized. Many of these fluorophores are commercially available in nucleotide-conjugated form, for example, to dCTP and/or dUTP.

The fluorophores to be used with each probe in the probe set should generally be chosen to allow them to be distinguished by fluorescence microscopy. For example, four mutually distinguishable fluorophores are SpectrumGreen, SpectrumOrange, SpectrumRed, and SpectrumAqua. Five-color FISH can be performed by using a mixed fifth probe, e.g., SpectrumAqua plus SpectrumOrange.

### 2. Chromosome enumeration probes

The probe sets described herein may comprise a chromosome enumeration probe, as defined above. The chromosome enumeration probe may have a hybridization site on chromosome 10, for example, the CEP10 probe (Vysis Abbott Molecular, Des Plaines, IL, USA).

### 3. Kits

The invention relates to kits comprising probe sets of the invention. The kits may also comprise other reagents useful for carrying out FISH assays, such as proteases (e.g., proteinase K or pepsin), buffers (e.g., sodium citrate), and/or chemical agents for sample pretreatment such as sodium thiocyanate.

### 4. Compositions

The invention provides compositions comprising probe sets of the invention, wherein the probes of the probe sets are distinguishably labeled. The compositions can be aqueous compositions and can comprise additional substances such as buffers (e.g., Tris, sodium bicarbonate, MOPS, HEPES), salts, and/or chelation agents (e.g., EDTA, EGTA).

### E. Assay methods

The invention provides methods of assaying for deletion of a tumor suppressor by FISH. The probe sets that can be used in such methods include the probe sets prepared as in section C and the probe sets of section D, above. In some embodiments, the probe sets have hybridization sites positioned with respect to boundary zones as discussed in sections C.2(b) and C.2(c).

Generally speaking, the methods of assaying for deletion of a tumor suppressor by FISH comprise steps of performing FISH with a probe set. The probe set comprises at least a first flanking probe that hybridizes to a position centromeric to the tumor suppressor gene, at least a second flanking probe that hybridizes to a position telomeric to the tumor suppressor gene, and at least one target probe that hybridizes to the tumor suppressor gene. FISH signals from flanking probes and target probe(s) are enumerated. At least one artifactual deletion frequency is provided; this serves as a negative control. It is possible for the artifactual deletion frequency to have been determined previously with control samples that do not comprise cells with a deletion of the tumor suppressor gene, and for the artifactual deletion frequency to be re-used in multiple assays. Based on the enumerated FISH signals, at least one apparent deletion frequency is determined. Whether the sample comprises cells with a deletion of the tumor suppressor gene is determined based on whether the apparent deletion frequency is significantly greater than the artifactual deletion frequency. The artifactual deletion frequency to which the apparent deletion frequency is compared should match in terms of whether the deletion frequencies are hemizygous or homozygous, and which probe hybridization sites are affected by the deletion.

### 1. Samples

The samples used in the methods of assaying for deletion of a tumor suppressor are by FISH are cellular samples. In some embodiments, the samples are chemically fixed using a preservative such as formalin, ethanol, formaldehyde, paraformaldehyde-glutaraldehyde, or a combination of sodium cacodylate, formalin, and glutaraldehyde and embedded in an inert solid material such as paraffin or a frozen tissue matrix, e.g., optimal cutting temperature (OCT) compound. In some embodiments, the samples comprise cells of which at least 50%, 60%, 70%, 80%, or 90% are non-metaphase (e.g., interphase) cells. See, e.g., Wick MR, Mills NC, Brix WK. Tissue Procurement, Processing, and Staining Techniques. Chapter 1. p. 1-10. Diagnostic Histochemistry edited by Wick MR. Cambridge University Press 2008.)

Generally, the samples are provided with a solid support (e.g., a paraffin matrix in which the sample is embedded, and/or a glass slide or wall of a micro titer well).

### 2. Performing FISH

The deletion assay methods of the invention involve fluorescence in situ hybridization (FISH). The term "in situ hybridization" generally refers to hybridization of a nucleic acid probe to a nucleic acid target that is part of a cytological or histological preparation. Typically, FISH methods involve the following steps: (a) providing a sample fixed to a solid support as discussed above; (b) treatment of the sample to increase accessibility of probe DNA to target DNA, e.g., with chemical or protease treatments (e.g., 10 mM citrate buffer pH 6.0 with 8% sodium thiocyanate; 0.2N HCl; or proteinase K at 25 µg/ml or pepsin at 750 U/ml), (c) contacting the tissue or material containing the target DNA with labeled probes of a probe set to form specific hybridization complexes, (d) post hybridization washes of the complexes to selectively remove probes that are not specifically hybridized to the target, and (e) detection of FISH signals from probes that have formed hybridization complexes with target DNA molecules. Such methods are described in a number of sources, including: Gall and Pardue, Methods of Enzymology 1981; 21:470-480; Henderson, International Review of Cytology, 1982; 76:1-46; Angerer, et al., in Genetic Engineering: Principles and Methods (Setlow and Hollaender, Eds.) 1985; vol. 7, pp. 43-65, Plenum Press, New York; and Varella-Garcia M et al., EGFR fluorescence in situ hybridisation assay: guidelines for application to non-small-cell lung cancer, J. Clin. Pathol. 2009; 62:970-977.

In some embodiments, treating the nuclei with proteolytic enzymes such as proteinase K or trypsin can mitigate non-specific binding of labeled DNA to protein in intact fixed nuclei. Also, image capture, using equipment such as a CCD (Charge Coupled Device). Also, image capture, using equipment such as a CCD (Charge Coupled Device) camera can be coupled with processing of images using image-processing software to reduce the background fluorescence arising from nonspecific hybridization. Furthermore, cross hybridization of repeat sequences in the probe with those in the genome can lead to complicating fluorescent signals. A Cot-1 DNA suppression step can be included in the hybridization protocols to ameliorate this problem. Because nucleic acids in the Cot-I fraction are characterized by containing highly repetitive sequences (e.g., Alu sequences, α-satellite, and β-satellite sequences), these nucleic acids bind to repeat sequences in the genome, thereby blocking binding of probes to such sequences (see, e.g., Benjamin Lewin, Genes V, 1994, Oxford University Press). In addition, probes depleted of repeat sequences may be prepared by physical removal or direct synthesis; *see, e.g.,* Rogan et al., Sequence-Based Design of Single-Copy Genomic DNA Probes for Fluorescence In Situ Hybridization, Genome Res. 2001; 11:1086-1094, Navin et al., PROBER: oligonucleotide FISH probe design software, Bioinformatics 2006; 22:2437-2438, and U.S. Application Publication No. 2003/0022166 (Collins et al.).

The following is an example of a procedure for preparing samples and performing FISH. Tissues can be fixed with a fixative such as formalin and then embedded in paraffin. Sections are then cut using a microtome and are applied to a microscope slide. Samples can be prepared from biopsies or other sources, depending on the type of cancerous or precancerous cells to be analyzed; for example, the source can be chosen from prostate, breast, melanoma, and other solid tumors: needle biopsy samples, fine needle aspirate biopsies, radical prostatectomies, metastatic samples (e.g., from bone or lymph node), cytology preparations (from body fluids such as urine or ascites), and circulating tumor cells isolated from peripheral blood. In some embodiments, specimens can be prepared by fixation of cells in ethanol or methanol:acetic acid combined with cytocentrifugation, thin layer deposition methods (e.g. ThinPrep, Cytyc Corp.), smears, or pipetting onto microscope slides.

Any suitable in situ hybridization method can be used. Prior to in situ hybridization, chromosomal DNA contained within the cell each are denatured. Denaturation typically is performed by incubating in the presence of high pH, heat (e.g., temperatures from 70 °C to 95 °C, for example, 83 °C), organic solvents such as formamide and tetraalkylammonium halides, or combinations thereof. For example, chromosomal DNA can be denatured by a combination of temperatures above 70 °C (e.g., 73 °C or 83 °C, incubated for about 5 minutes) and a denaturation buffer containing 70% formamide and 2X SSC (0.3M sodium chloride and 0.03 M sodium citrate). Denaturation conditions typically are established such that cell morphology is preserved. For example, chromosomal probes can be denatured by heat, e.g., by heating the probes to about 73 °C or 83 °C for about five minutes.

An example of a processing procedure for slide-mounted FFPE samples is shown in the following table.

| | **Action** | **Temperature** | **Incubation Time** |
|---|---|---|---|
| | **DAY 1** | | |
| 1 | Xylene, 3x | RT | 10 Minutes each |
| 2 | 100% EtOH, 2x | RT | 5 Minutes each |
| 3 | 85%,70% EtOH, dH2O | RT | 2 Minutes each |
| 4 | Incubate in the 0.2N HCL | RT | 20 Minutes each |
| 5 | Incubate in pre-warmed 1M NaSCN | 80°C | 10 Minutes each |
| 6 | 2xSSC | RT | 2 Minutes each |
| 7 | dH20 | RT | 5 Minutes each |
| 8 | Incubate in the 0.2N HCL | RT | 2 Minutes each |
| 9 | Incubate in pre-warmed 0.01 N HCL+Pepsin | 37°C | 10 Minutes each |
| 10 | dH2O | RT | 5 Minutes each |
| 11 | 70%,85%,and 100% EtOH | RT | 2 Minutes each |
| 12 | Air Dry Slides | | |
| 13 | Assess tissue morphology using phase contrast microscopy | | |
| 15 | Vortex then briefly centrifuge probe | | |
| 16 | Apply probe mix to slide and immediately apply coverslip | | |
| 17 | Seal the coverslip with rubber cement | | |
| 18 | Place the slide on a Thermobrite® | 83°C | 5 Minutes |
| 19 | Target and probe DNA hybridization | 37°C | 16-24 Hours |
| | | | |

| | **DAY 2** | | |
|---|---|---|---|
| 20 | Using forceps, carefully remove rubber cement | | |
| 21 | 0.4xSSC/0.3% NP40 | 72°C | 2 Minutes |
| 22 | 2xSSC | RT | 2 Minutes |
| 23 | Apply Dapi counterstain to the target area | | |
| 24 | Coverslip using 24x50 | | |

In some embodiments, such as when the probes of the probe set are provided in double-stranded form, the probes of the probe set may also be denatured prior to in situ hybridization. Denaturation conditions such as those described above can be used.

After removal of denaturing chemicals or conditions, probes are annealed to the chromosomal DNA under hybridizing conditions. "Hybridizing conditions" are conditions that facilitate annealing between a probe and target chromosomal DNA. Hybridization conditions vary, depending on the concentrations, base compositions, complexities, and lengths of the probes, as well as salt concentrations, temperatures, and length of incubation. For example, in situ hybridizations can be performed in hybridization buffer containing 1X to 2X SSC, 50-55% formamide, a hybridization accelerant (e.g. 10% dextran sulfate), and unlabeled blocking DNA to suppress non-specific hybridization. In general, hybridization conditions, as described above, include temperatures of 25 °C to 55 °C, and incubation lengths of 0.5 hours to 96 hours. In some embodiments, hybridization can be performed at a temperature from 32 °C to about 45 °C for 2 to 16 hours.

Non-specific binding of chromosomal probes to DNA outside of the target region can be removed by a series of washes with a salt solution. Temperature and concentration of salt in each wash depend on the desired stringency. For example, for high stringency conditions, washes can be carried out at about 65 °C to about 80 °C, using 0.2X to 2X SSC, and 0.1 % to 1% of a non-ionic detergent such as NP-40. Stringency can be lowered by decreasing the temperature of the washes or by increasing the concentration of salt in the washes. The hybridization of the probes to the tissue sample can be performed manually, or with the assistance of instruments, such as the ThermoBrite hybridization oven, the VP 2000 Processor, or the XMatrix(TM) processing instrument (all available commercially from Abbott Molecular, Inc.).

Detection of FISH signals from a hybridized and washed sample can be accomplished to allow subsequent analysis in real time, such as by an operator who views fluorescence directly at a microscope, or to allow later analysis, by recording images. Detection generally involves the use of an appropriate source of excitation light for each fluorophore in use in the assay (e.g., light passed through an appropriate filter, or light from a laser of an appropriate wavelength); fluorescently emitted light is then passed through an appropriate filter, and it can be viewed directly by an operator and/or photographed with a camera, such as a film-based or digital camera, which may be connected to a computer.

### 3. Enumeration

The FISH signals of a plurality of nuclei in the sample are enumerated. In some embodiments, the plurality of nuclei comprises at least 50, 75, or 100 nuclei. Enumeration can be achieved, for example, by making a list containing an entry for each examined nucleus and how many of each of the FISH signals it contained, and/or by counting the number of nuclei that had each observed combination of FISH signal quantities. In some embodiments, such a list can contain possible but unobserved combinations of FISH signal quantities as well. The list can be reorganized by making entries for each observed combination of FISH signal quantities, accompanied by their frequency (see, e.g., Table 2 below). The information obtained by enumerating FISH signals is to be interpreted in view of at least one artifactual deletion frequency, discussed below.

### 4. Artifactual deletion frequency

The deletion assay methods comprise providing at least one artifactual deletion frequency. A nucleus can lack a FISH signal from a target probe for reasons other than a deletion; for example, in the case of a sectioned sample, including formalin-fixed, paraffin embedded samples, some nuclei in the sample may be truncated. It is also possible that the target hybridization site(s) in a fraction of the nuclei may not be sufficiently accessible during the hybridization step of the FISH procedure. In addition, the frequency with which FISH signals are observed can be influenced by the quality of and effects of aging on the tissue preparations being studied. Thus, not every nucleus lacking a FISH signal from the target probe is indicative of an actual genetic deletion.

Therefore, in order to mitigate the potential for false positives (in which a deletion is called erroneously), at least one artifactual deletion frequency is provided in order to distinguish between results from a sample indicative of cells having a deletion of the target of the assay, and results from a sample in which the causes of absent target probe signals are likely other than the underlying genotype, such as the technical sources of error discussed above.

An artifactual deletion frequency can be determined based on a control assay, using samples of cells known not to have a deletion of the target gene. The accuracy of the artifactual deletion frequency determined from control samples may be optimized relative to a test sample by using control samples of cells matched closely to the test sample of cells with respect to the sample preparation method. For example, non-neoplastic tissue samples such as benign hyperplasias of the same tissue type as the precancerous/cancerous/potentially cancerous samples to be tested for loss of a tumor suppressor gene can serve as controls. In the case of assays on prostate cancers, non-neoplastic tissue samples can be obtained from patients without cancer who are undergoing surgery solely for benign prostate hyperplasia (BPH) for control purposes. The artifactual deletion frequency is also influenced by other biological variables affecting both FISH signals and relative nuclear volume in tumor sections. The condensation of the DNA, the size and geometry of the nucleus, the thickness of the sample sections, the assay design (the signal distance in 3 or 4 color FISH assay depends partially on the genomic distance of the probes), and the growth rate and ploidy status (higher likelihood of higher levels of ploidy convey larger volume nuclei). In addition to these nuclear variables, the actual FISH signal itself (the "spot size") may occupy more three-dimensional space in decondensed nuclear chromatin (typical open DNA packing of expressed genes) in comparison to condensed regions of the genome (DNA of repressed genes for example). Moreover, individual FISH signals may be more difficult to interpret if DNA replication has already taken place, since G2 nuclei exhibit "doublet" or paired spot counts due to the duplication of the cell's DNA. These Doublets are physically linked paired spots, i.e. touching or linked by a thread, or adjacent (with a gap smaller than the diameter of the largest signal or spot). In some embodiments, scoring criteria can be adjusted to ensure that this effect does not distort the results of the assays (see Varella-Garcia M et al., EGFR fluorescence in situ hybridisation assay: guidelines for application to non-small-cell lung cancer, J Clin Pathol. 2009; 62:970-977). This can include counting physically linked paired spots and/or adjacent spots as one signal only. Spots that are adjacent but separated by at least the diameter of the largest signal can be counted as separate signals. On the other hand, prior to DNA replication each signal shows a single dot-like hybridization signal ("singlet") and these are also counted as one signal only. Several of these variables such as ploidy, nuclear size, and chromatin condensation can vary between normal control samples and tumor specimens. Thus, these factors may be difficult to fully account for in negative controls used to establish an artifactual deletion frequency for interphase FISH assays; this can be of particular importance in samples with low-tumor cell content or additional subclonal genomic changes. Thus, it can be advisable to use a relatively stringent statistical threshold for calling deletions, discussed further below.

In some embodiments, at least two, or more, artifactual deletion frequencies are provided. For example, artifactual deletion frequencies for hemizygous and homozygous deletions can be provided. Also, separate artifactual deletion frequencies can be provided for deletions that lead to absence of FISH signals from different combinations of probes, for example, deletions that affect only the target probe, deletions that affect the target probe and the centromeric flanking probe closest to the target probe, deletions that affect the target probe and the telomeric flanking probe closest to the target probe, and deletions that affect the target probe, the centromeric flanking probe closest to the target probe, and the telomeric flanking probe closest to the target probe. In embodiments in which a probe set comprising more than two flanking probes are used, artifactual deletion frequencies can be provided for each probe within a given set so that deletions affecting the flanking probes more distant from the target probe can be considered as well.

An advantage of providing separate artifactual deletion frequencies is that the FISH signals missing due to sources of error, such as truncation events, can be more variable from nucleus to nucleus than the genotype of a clonal population, such that the artifactual deletion frequency for any given type of deletion (e.g., a hemizygous deletion that affects the target probe and the telomeric flanking probe closest to the target probe) is lower than it would be if at least one centromeric flanking probe were not also used in the assay, because some of the sources of error such as truncation events may affect the at least one centromeric flanking probe as well.

Reduction of the artifactual deletion frequency can be very helpful depending on the cell type under analysis, in that some cell types are especially prone to truncation artifacts. For example, in a recent study of truncation losses of signals by interphase FISH in 5-micron histological sections, it was found that about 20% of normal bone marrow nuclei exhibited signal losses due to truncations. In contrast, ∼60% of nuclei in normal liver sections exhibited truncation losses (Wilkens L et al. 2005. Standardised fluorescence in situ hybridisation in cytological and histological specimens. Virchows Arch 2005; 447: 586-592). This variation in false positive rates for detecting losses is thought to be due to the cutting artifacts of truncation that have an increasing influence when the much larger, irregularly shaped liver cell nuclei are more frequently truncated during the preparation of 5-micron sections.

### 5. Determining at least one apparent deletion frequency

The information obtained by enumerating FISH signals is analyzed to determine at least one apparent deletion frequency. An apparent frequency is an observation of how often the target probe is missing in a sample. It should be noted that this step may occur with any timing relative to the step of providing at least one artifactual deletion frequency. Much as with artifactual deletion frequencies, in some embodiments, at least two, or more, apparent deletion frequencies are determined. In the interests of efficiency, it is generally advisable to determine apparent deletion frequencies and provide artifactual deletion frequencies in pairs amenable to comparison (e.g., artifactual and apparent deletion frequencies for a hemizygous deletion that affects the target probe and the telomeric flanking probe closest to the target probe). In any event, in order to perform the next step, at least one apparent deletion frequency must be for the same type of deletion event as at least one artifactual deletion frequency.

### 6. Detection of tumor suppressor gene deletions

Whether the sample comprises cells with a deletion is determined based on whether at least one apparent deletion frequency is significantly greater than at least one artifactual deletion frequency. The threshold for significance can be chosen according to the desired level of specificity versus sensitivity; common values include a *p*-value less than or equal to 0.05 according to a statistical test such as the *t*-test (one or two-tailed), or a difference of at least three standard deviations from the artifactual deletion frequency. In some embodiments, a significance threshold such as the artifactual deletion frequency plus at least 2.5, 3, or 3.5 standard deviations is used to determine whether the at least one apparent deletion frequency is significantly greater than at least one artifactual deletion frequency.

As discussed above, multiple artifactual deletion frequencies can be provided, and multiple apparent deletion frequencies can be determined. Thus, this step can comprise determining whether multiple types of deletion are present. For example, the presence of two sets of FISH signals from the flanking and target probes indicates that a nucleus does not have a deletion of the target tumor suppressor (schematized in Fig. 2B, left diagram). Absence of one or both of the target probe FISH signals indicates an apparent hemizygous (Fig. 2B, center) or homozygous (Fig. 2B, right) deletion, respectively. Information about the size of the apparent deletion can be obtained from whether one or more of the flanking probes are also absent. The examples of Fig. 2B right and center are indicative of relatively small deletions in which only the target probe was affected.

Thus, in some embodiments, the methods comprise determining whether the sample comprises cells with a deletion chosen from deletions that affect only the target probe, deletions that affect the target probe and the centromeric flanking probe closest to the target probe, deletions that affect the target probe and the telomeric flanking probe closest to the target probe, and deletions that affect the target probe, the centromeric flanking probe closest to the target probe, and the telomeric flanking probe closest to the target probe.

In addition to the simple deletion configurations shown in Figure 2B, more complex patterns involving gain or loss of signals may be seen because of additional rearrangements close to the regions containing the flanking control probes. For example, some of the *PTEN* gene losses observed in advanced prostate cancer appear to have complex losses involving genes closely linked to *PTEN* that are associated with deletion events (discussed below). In addition, complex signal configurations bearing additional spots may also arise from complex chromosomal gains due to unbalanced translocations, polysomies, or polyploidy. Any pattern differing from the simple patterns observed in normal nuclei is also usually considered abnormal if it appears in a significant proportion of cells. Careful evaluation of the number and location of signals in aberrant patterns can provide valuable information of underlying chromosomal change.

### 7. Boundary zones

One or more of the flanking probes used in the assays can have a hybridization site in a boundary zone or distal to the tumor suppressor gene relative to a boundary zone. In some embodiments, the at least one first flanking probe hybridizes to a position within or centromeric to a first boundary zone centromeric to the tumor suppressor gene, and/or the at least one second flanking probe hybridizes to a position within or telomeric to a second boundary zone telomeric to the tumor suppressor gene. Use of flanking probes with hybridization sites so positioned can be helpful because the a hybridization site of a flanking probe positioned in this way should be less likely to be deleted than if it were proximal to the tumor suppressor gene relative to the boundary zone. Use of such a probe set can raise the frequency of deletions which give patterns as in the center and right of Fig. 2B. These patterns have signals from flanking probes that hybridize centromerically and telomerically to the (presumably partly or completely deleted) tumor suppressor gene, and these patterns are less likely to arise repeatedly through truncation artifacts in which a section of the volume of the nucleus is ablated, because it would be necessary for the chromosome to have been arranged in a manner such that a loop of chromatin containing the tumor suppressor gene but neither flanking probe hybridization site extended into the ablated nuclear volume. Boundary zones and positions of hybridization sites relative thereto are discussed in greater detail in section C.2 above; that discussion is relevant here as well.

### F. Assays for distinguishably detecting small and large deletions

It is possible that cells in which both copies of a tumor suppressor gene have been deleted have undergone two separate deletion events of different sizes. The invention relates to methods of distinguishably detecting deletion events of different sizes.

### 1. Probe sets

In these methods, a probe set of at least four probes is used. The probe set comprises at least one target probe that hybridizes to the tumor suppressor gene at least a first flanking probe that hybridizes to a position centromeric to the tumor suppressor gene, at least a second flanking probe that hybridizes to a position telomeric to the tumor suppressor gene, and at least one of at least a third flanking probe that hybridizes to a position centromeric to the hybridization site of the first flanking probe or at least a fourth flanking probe that hybridizes to a position telomeric to the hybridization site of the second flanking probe. The probe set can additionally comprise a centromeric or pericentromeric probe.

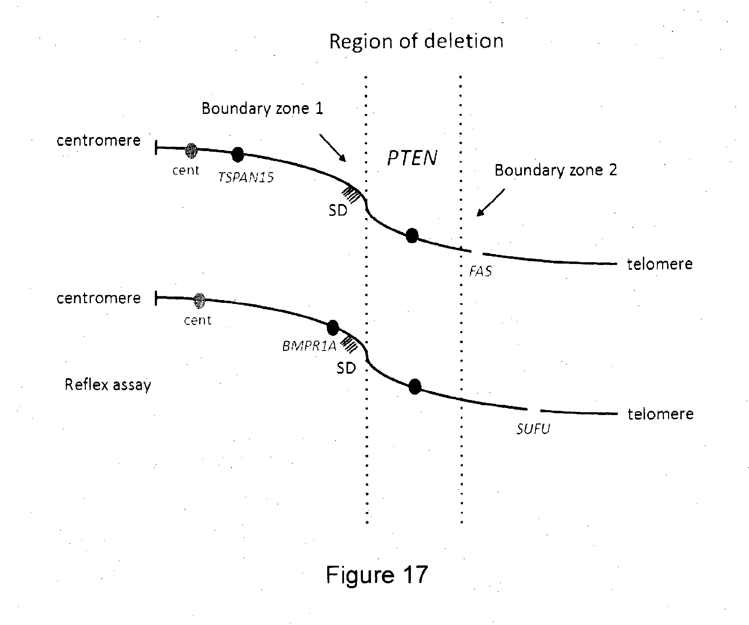

In some embodiments, the probe set comprises two subsets which comprise partially overlapping probes. The two subsets can be used in a two-part assay in which at least one differently positioned flanking probe is used in the second or reflex part of the assay. For example, Figure 17 provides an illustration of one possible configuration of probes for a two part assay for small and large *PTEN* deletions in which a centromeric probe, a *TSPAN15* probe, a *PTEN* probe, and a *FAS* probe are used in the first part, and a centromeric probe, a *BMPR1A* probe, a *PTEN* probe, and a *SUFU* probe are used in the second part. If a deletion exceeds the region bounded by the vertical dotted lines in Fig. 17, one or more of the flanking probes should be affected; very large deletions would be indicated when the *TSPAN15* or *SUFU* probes are affected. For example, it is possible for a deletion to result in the loss of the entire region from *PTEN* to the telomere, which would affect the *PTEN, FAS,* and *SUFU* probes. More generally, the probe set can comprise at least two subsets comprising partially overlapping probes, wherein the first subset comprises at least one target probe, at least one centromeric flanking probe, at least one telomeric flanking probe, and optionally at least one probe with a centromeric or pericentromeric hybridization site, and the second subset comprises the at least one target probe, at least one centromeric flanking probe, at least one telomeric flanking probe, and optionally at least one probe with a centromeric or pericentromeric hybridization site, with at least one of the at least one centromeric flanking probe and the at least one telomeric flanking probe of the second subset being different from its counterpart in the first subset.

### 2. Enumeration; artifactual and apparent deletion frequencies

The steps of enumerating FISH signals, providing artifactual deletion frequencies, and determining apparent deletion frequencies are similar to those discussed in sections E.3-E.5 above, except that there are more probes to enumerate, and artifactual and apparent frequencies for more types of deletions can be provided and determined, respectively. For example, at least one first artifactual deletion frequency can be provided for deletions of the tumor suppressor gene with endpoints between the at least first and at least second flanking probes (i.e., the flanking probes closest to the target probe in the centromeric and telomeric directions), and at least one second artifactual deletion frequency can be provided for deletions of the tumor suppressor gene with endpoints between the two most distal flanking probes, wherein at least one of the endpoints is not between the at least first and at least second flanking probes. The above discussion is applicable regardless of whether the at least three flanking probes are hybridized to the sample(s) together or in a two-part assay.

Likewise, at least one first apparent deletion frequency can be determined for deletions of the tumor suppressor gene with endpoints between the at least first and at least second flanking probes, and at least one second apparent deletion frequency can be determined for deletions of the tumor suppressor gene with endpoints between the two most distal flanking probes, wherein at least one of the endpoints is not between the at least first and at least second flanking probes.

### 3. Determining whether the sample comprises cells with a small or large deletion

Whether the sample comprises cells with a deletion of the tumor suppressor gene that affects the hybridization site of any of the flanking probes used is determined based on whether the relevant apparent deletion frequency is significantly greater than the relevant artifactual deletion frequency, as described above in section E.6.

A sample in which the target probe has an apparent hemizygous or homozygous deletion frequency significantly greater than the appropriate artifactual deletion frequency is determined to comprise a small deletion or deletions only (not affecting any flanking probes) if none of the flanking probes have apparent deletion frequencies significantly greater than the appropriate artifactual deletion frequency.

If the target probe and at least one flanking probe have apparent deletion frequencies significantly greater than the appropriate artifactual deletion frequency, then it is likely that a large deletion is present.

It should be noted that the possibility of an unusual pair of small deletions in which only the hybridization of a flanking probe is deleted on one copy of chromosome 10 and only the hybridization site of the target probe is deleted on the other copy of chromosome 10 can generally be ruled out based on the tendency of FISH signals from the same chromosome to be near one another; thus, in the case of a hemizygous large deletion, the FISH signals from the unaffected chromosome should be near one another.

It is possible for both small and large deletions to be present; in these cases, the target probe has an apparent homozygous deletion frequency significantly greater than the corresponding artifactual homozygous deletion frequency, and at least one flanking probe has an apparent hemizygous deletion frequency significantly greater than the corresponding artifactual Hemizygous deletion frequency. In cases in which the large deletion has resulted in the loss of two flanking probe hybridization sites (e.g., a loss of the entire region from the tumor suppressor to the telomere which affects two flanking probes with hybridization sites telomeric to the tumor suppressor), then two flanking probes would have an apparent hemizygous deletion frequency significantly greater than the corresponding artifactual hemizygous deletion frequency.

In some embodiments, homozygous deletion of the tumor suppressor gene wherein there is at least one large deletion is indicative of a metastasizing or metastatic tumor; a metastasizing tumor is able to give rise to at least a second tumor at another location in the body, and the second tumor is a metastatic tumor. In some such embodiments, one copy of the tumor suppressor gene is missing due to a small deletion and one copy is missing due to a large deletion. In some embodiments, both copies of the tumor suppressor gene are missing due to large deletions. In some embodiments, there is at least one large deletion which affects at least two flanking probe hybridization sites located telomeric to the tumor suppressor. In some embodiments, the more distal (with respect to the tumor suppressor gene) of said at least two flanking probe hybridization sites is at least 5, 7.5, 10, or 12.5 Mb from the tumor suppressor gene.

In some embodiments, the method for detecting small and large deletions comprises performing FISH on a first cellular sample comprising a plurality of cells with a first probe subset comprised by the probe set and performing FISH on a second cellular sample from the same individual as the first cellular sample comprising a plurality of cells with a second probe subset comprised by the probe set; the tumor suppressor gene is *PTEN;* the first probe subset comprises a flanking probe that hybridizes to TSPAN15 and a flanking probe that hybridizes to *FAS*; and the second probe subset comprises a flanking probe that hybridizes to *BMPR1A* or *WAPAL* (it being understood that a probe that hybridizes to one of *BMPR1A* or *WAPAL* may also hybridize to the other, as they are neighboring genes) and a flanking probe that hybridizes to *SUFU.* When a *PTEN* deletion is detected in this method, analysis of whether at least one of the hybridization sites of the flanking probes is deleted along with PTEN can be used to determine whether the deletion is a large deletion.

### 4. Boundary zones

As in the methods of section E, in some embodiments, the at least one first flanking probe hybridizes to a position within or centromeric to a first boundary zone centromeric to the tumor suppressor gene, and/or the at least one second flanking probe hybridizes to a position within or telomeric to a second boundary zone telomeric to the tumor suppressor gene.

It is also possible for the third and/or fourth flanking probes to be positioned within a boundary zone or distal to the tumor suppressor gene relative to a boundary zone. That is to say, in some embodiments, the at least one third flanking probe hybridizes to a position centromeric to a first distal boundary zone centromeric to the hybridization site of the at least one first flanking probe, and/or the at least one fourth flanking probe hybridizes to a position telomeric to a second distal boundary zone telomeric to the hybridization site of the at least one second flanking probe.

Boundary zones and positions of hybridization sites relative thereto are discussed in greater detail in section C.2 above; that discussion is relevant here as well.

### G. Methods for assay optimization

Described herein are methods of optimizing a FISH-based assay for deletion of a tumor suppressor gene. In these methods, a plurality of candidate probe sets is provided, and each probe set is used to perform FISH on at least one sample comprising a plurality of cells comprising a euploid number of intact copies of the tumor suppressor gene. FISH signals are enumerated, and an artifactual deletion frequency is determined. A probe set determined to have a favorable artifactual deletion rate is selected from the candidate probe sets for use in the optimized FISH-based assay for deletion of a tumor suppressor gene.

### 1. Candidate probe sets

At least two, at least three, at least four, at least five, or more candidate probe sets may be provided. The candidate probe sets can have some probes in common; for example, at least two candidate probe sets can comprise at least one identical target probe, and/or at least one identical flanking probe. Of course, at least one probe should differ between each candidate set. In some embodiments, at least one, at least two, at least three, or at least four of the flanking probes are positioned within a boundary zone or distal to the tumor suppressor gene relative to a boundary zone, to which the probes may be near.

### 2. Artifactual deletion frequency determination

An artifactual deletion frequency is determined for each candidate probe set by performing FISH on at least one sample, comprising a plurality of cells comprising a euploid number of intact copies of the tumor suppressor gene. An artifactual deletion frequency is determined by enumerating FISH signals as described above. In some embodiments, FISH is performed with each probe set on a plurality of samples, such as at least two, at least three, at least four, or at least five, in order to allow a more statistically rigorous comparison of the measured artifactual deletion frequencies.

### 3. Selection of a probe set with a favorable artifactual deletion frequency

A probe set that was found to give a favorable artifactual deletion frequency is selected for use in an optimized assay. The artifactual deletion frequency of the selected probe set may be in at least the 60th, 70th, 80th, 90th, or 95th percentile, it being understood that higher percentiles indicate better performance (i.e., lower artifactual deletion frequency).

### 4. Use of samples with deletions

FISH may also be performed with each candidate probe set, or with a subset of the candidate probe sets that have been found to give favorable artifactual deletion frequencies, on a plurality of cellular samples comprising a plurality of cells comprising a homozygous or hemizygous deletion of the tumor suppressor gene. FISH signals are enumerated, and an apparent deletion frequency is determined, as described above.

### (a) Sensitivity

As the samples are known to comprise cells comprising a homozygous or hemizygous deletion of the tumor suppressor gene, sensitivity values are determined based on how many of the plurality of samples were determined to have an apparent deletion frequency significantly greater than the artifactual deletion frequency determined for the candidate probe set. A standard deviation for the artifactual deletion frequency of a candidate probe set may be determined in the usual fashion as described above, provided that the artifactual deletion frequency was determined for at least three samples comprising a plurality of cells comprising a euploid number of intact copies of the tumor suppressor gene. Alternatively, a standard deviation of the artifactual deletion frequency may be estimated (e.g., based on artifactual deletion frequencies of an ensemble of candidate probe sets, or based on known standard deviations for other probe sets) for use in determining significance in order to assess sensitivity. Options for the threshold for determining significance are as discussed above.

### (b) Selection of a probe set in view of sensitivity value and artifactual deletion frequency

When sensitivity is determined for at least some of the candidate probe sets, a candidate probe set that had both a favorable sensitivity value and a favorable artifactual deletion frequency can be selected for use in the optimized assay. The artifactual deletion frequency and/or sensitivity values may be in at least the 60th, 70th, 80th, 90th, or 95th percentile, it being understood that higher percentiles indicate better performance (i.e., higher sensitivity or lower artifactual deletion frequency).

### H. Assay methods for characterization of proto-oncogenic and oncongenic loci

Tumor-suppressor genes, such as PTEN, are those that encode proteins that in one way or another inhibit cell proliferation. Loss of one or more of these "cellular brakes" contributes to the development of many cancers through release of cell cycles, making the loss event "oncogenic." Many proteins are generally recognized as being encoded by tumor-suppressor genes, including intracellular proteins, such as the p16 cyclin-kinase inhibitor; receptors for secreted hormones (e.g., tumor-derived growth factor β); checkpoint-control proteins that arrest the cell cycle if DNA is damaged or chromosomes are abnormal; proteins that promote apoptosis; and even enzymes that participate in DNA repair (cells that have lost the ability to repair errors, gaps, or broken ends in DNA accumulate mutations in many genes, including those that are critical in controlling cell growth and proliferation).

Since one copy of a tumor-suppressor gene may suffice to control cell proliferation, usually both alleles of a tumor-suppressor gene must be lost or inactivated in order to promote tumor development. Thus, oncogenic loss-of-function mutations in tumor-suppressor genes tend to act recessively. In many cancers, deletions or point mutations have occurred in one or more tumor-suppressor genes that prevent production of any protein or lead to production of a nonfunctional protein from the mutated tumor suppressor genes. However, even the loss of one copy of a tumor suppressor may predispose a cell to a second oncogenic event. For example, such a relationship appears to exist between *TMPRSS2-ERG* and *PTEN* loss, and a further connection with AKT. See Squire, JA, TMPRSS-ERG and PTEN loss in prostate cancer, Nat Genetics (2009) 41, 509-510. Aberrant ERG expression had been characterized as an oncogenic event in mice that is associated with *PTEN* loss. See Carver, BS, Tran J, Gopalan A, Chen Z, et al., Aberrant ERG expression cooperates with loss of PTEN to promote cancer progression in prostate. Nat Genetics (2009) 41, 619-624. Carver et al. suggested that, while the loss of one copy of *PTEN* may promote cell proliferation, the cell did not progress to invasive disease unless ERG was overexpressed. *See id.* Likewise, mice having a human ERG overexpression construct alone appear not to develop prostate tumors, but progeny from crosses with mice having *PTEN* deletions did develop tumors. See King JC, et al. Nat Genetics (2009) 41, 524-526.

Even with recent discoveries, remarkably little is actually known about such oncogenic events in prostatic carcinogenesis, though prostate cancer is the most common neoplasm and second leading cause of cancer mortality in North American men. See Carver, BS, Tran J, Gopalan A, Chen Z, et al., Aberrant ERG expression cooperates with loss of PTEN to promote cancer progression in prostate. Nat Genetics (2009) 41, 619-624. With regard to *PTEN* related oncogenic events, *ERG1* was observed in the prostate cancer (CaP) transcriptome, consistent with a role as an over-expressed proto-oncogene in malignant prostatic tissues. See Jemal A, et al., Cancer statistics, 2005. CA CancerJ Clin. 2005;55:10-30. And Tomlins et al., Recurrent fusion of TMPRSS2 and ETS transcription factor genes in prostate cancer, Science, 2005;310:644-648, described the first recurrent gene rearrangement in prostate cancer, which resulted in translocation of *ERG* to the promoter region of *TMPRSS2.* In addition, other genes were also shown to be translocated to androgen responsive or other strong promoters, as is now thought to underlie the mechanism of *ERG1* over-expression. *See id.* The gene fusions described to date involve the androgen-sensitive *TMPRSS2* gene and three aforementioned members of the *ETS* family of transcription factors (*ERG*, *ETV1*, and *ETV4).* The above studies begin with the detection of expression, and subsequently find those rearrangements that result in over-expression via deletion or other rearrangement.

Therefore, the identification of chromosomal rearrangements leading to deletions of tumor suppressors, or subsequent overexpression of proto-oncogenes (e.g., *ERG)* identified in prostate cancer, represents a new opportunity to understand epithelial tumors, which in general have been previously characterized only by nonspecific chromosomal aberrations.

In a recent study, the presence of a common deletion of approximately 2Mb of chromosome 21q22.2-3 was described as causing the *TMPRSS2:ERG* rearrangements discussed above, and as associated with disease progression. *See* Yoshimoto M, et al., Three-Color FISH Analysis of TMPRSS2/ERG Fusions in Prostate Cancer Indicates That Genomic Microdeletion of Chromosome 21 Is Associated with Rearrangement. Neoplasia (2006) 8, 465-469. In addition, break-apart three-color FISH was used to confirm that a deletion between TMPRSS2 and ERG on chromosome 21 was associated with gene fusion events.

Thus, a region of approximately 2Mb is deleted resulting in the overexpression of ERG. This common deletion results in effective in-frame fusion gene formation. However, there is little, if any, information available on the consequences of losing the 13 genes intervening TMPRSS2 and ERG on the progression of prostate cancer.

Little to nothing is actually known about the events leading to rearrangements of chromosome 21 to yield TMPRSS2-ERG fusions and consequent overexpression, or whether the fusions or overexpression are related to reduced PTEN expression due to genomic deletions. Almost all emphasis is on the fusion and consequent overexpression of ERG. Thus, the current invention may also be used to characterize the nature of subsequent events that relate to PTEN status.

Described herein are methods of assaying for deletion or other rearrangements of tumor suppressor(s), oncogene(s), proto-oncogene(s), or other genes of interest by FISH. These include methods for assaying specific target genes of interest in a chromosomal region commonly deleted when the TMPRSS2-ERG fusion gene is formed, that results in loss of ~2 Mb of DNA containing 13 genes (e.g. the 21q22.2 region containing ERG, ETS2, FSMG1, B3GALT5, HMGN1, DSCAM, BACE2, and TMPRSS2). The probe sets that can be used in such methods include the probe sets prepared as in sections C or E and the probe sets of section D above. In some methods, the probe sets have hybridization sites positioned with respect to boundary zones as discussed in sections C.2(b) and C.2(c).

Generally speaking, the methods of assaying for deletion of at least one gene of interest by FISH comprise steps of performing FISH with a probe set. The probe set comprises at least a first flanking probe that hybridizes to a position centromeric to the gene(s) of interest, at least a second flanking probe that hybridizes to a position telomeric to the gene(s) of interest, and at least a target probe or probes that hybridize to the gene(s) of interest. FISH signals from flanking probes and target probe(s) are enumerated. At least one artifactual deletion frequency caused by nuclear truncations is provided; this serves as a negative control. It is possible for the artifactual deletion frequency to have been determined previously with control samples that do not comprise cells with a deletion or rearrangements of the gene(s) of interest, and for the artifactual deletion frequency to be re-used in multiple assays. Based on the enumerated FISH signals, at least one apparent deletion frequency is determined. Whether the sample comprises cells with a deletion of the gene(s) of interest is determined based on whether the apparent deletion frequency is significantly greater than the artifactual deletion frequency. The artifactual deletion frequency to which the apparent deletion frequency is compared should match in terms of whether the deletion frequencies are hemizygous or homozygous, and which probe hybridization sites are affected by the deletion.

### 1. Samples

The samples used in the methods of assaying for deletion of at least one gene of interest by FISH, are cellular samples. The samples may be chemically fixed using a preservative such as formalin, ethanol, formaldehyde, paraformaldehyde-glutaraldehyde, or a combination of sodium cacodylate, formalin, and glutaraldehyde and embedded in an inert solid material such as paraffin or a frozen tissue matrix, e.g., optimal cutting temperature (OCT) compound. In some embodiments, the samples comprise cells of which at least 50%, 60%, 70%, 80%, or 90% are non-metaphase (e.g., interphase) cells. See, e.g., Wick MR, Mills NC, Brix WK. Tissue Procurement, Processing, and Staining Techniques. Chapter 1. p. 1-10. Diagnostic Histochemistry edited by Wick MR. Cambridge University Press 2008.

Generally, the samples are provided with a solid support (e.g., a paraffin matrix in which the sample is embedded, and/or a glass slide or wall of a micro titer well).

### 2. Performing FISH

The deletion assay methods described herein involve fluorescence in situ hybridization (FISH). The term "in situ hybridization" generally refers to hybridization of a nucleic acid probe to a nucleic acid target that is part of a cytological or histological preparation. Typical FISH methods are discussed in Section F.2 above.

Examples of procedures for preparing samples and performing FISH are well known in the art and discussed in section F. As discussed, samples can be prepared from many sources, depending on the type of cancerous or precancerous cells to be analyzed; for example, the source can be chosen from prostate, breast, melanoma, and other solid tumors. Samples can be obtained from needle biopsies, fine needle aspirate biopsies, radical prostatectomies, metastatic samples (e.g., from bone or lymph node), cytology preparations (from body fluids such as urine or ascites), and circulating tumor cells isolated from peripheral blood using standard preparations for fixation of cells in ethanol or methanol: acetic acid combined with cytocentrifugation, thin layer deposition methods (e.g. ThinPrep, Cytyc Corp.), smears, or pipetting onto microscope slides.

Suitable in-situ hybridization methods can be used and are discussed in Section E2. Prior to in situ hybridization, chromosomal DNAs contained within the cells are denatured, typically by incubating in the presence of high pH, heat (e.g., temperatures from 70 ºC to 95 ºC), organic solvents such as formamide and tetraalkylammonium halides, or combinations thereof. Denaturation conditions typically are established such that cell morphology is preserved. For example, chromosomal DNA can be denatured by a combination of temperatures above 70 ºC (e.g., 73 ºC) and a denaturation buffer containing 70% formamide and 2X SSC (0.3M sodium chloride and 0.03 M sodium citrate). Alternatively, e.g., chromosomal DNA obtained from Fine needle biopsy samples may be denatured at 83 ºC for 5 minutes.

In some methods, such as when the probes of the probe set are provided in double-stranded form, the probes of the probe set may also be denatured prior to in situ hybridization. Denaturation conditions such as those described above can be used.

After removal of denaturing chemicals or conditions, probes are annealed to the chromosomal DNA under hybridizing conditions. "Hybridizing conditions" are conditions that facilitate annealing between a probe and target chromosomal DNA. Hybridization conditions vary, depending on the concentrations, base compositions, complexities, and lengths of the probes, as well as salt concentrations, temperatures, and length of incubation. For example, in situ hybridizations can be performed in hybridization buffer containing 1X to 2X SSC, 50-55% formamide, a hybridization accelerant (e.g. 10% dextran sulfate), and unlabelled blocking DNA to suppress non-specific hybridization. In general, hybridization conditions, as described above, include temperatures of 25 °C to 55 °C, and incubation lengths of 0.5 hours to 96 hours. In some embodiments, hybridization can be performed at a temperature from 32 °C to about 45 °C for 2 to 16 hours.

Non-specific binding of chromosomal probes to DNA outside of the target region can be removed by a series of washes with a salt solution. Temperature and concentration of salt in each wash depend on the desired stringency. For example, for high stringency conditions, washes can be carried out at about 65 °C to about 80 °C, using 0.2X to 2X SSC, and 0.1 % to 1% of a non-ionic detergent such as NP-40. Stringency can be lowered by decreasing the temperature of the washes or by increasing the concentration of salt in the washes. The hybridization of the probes to the tissue sample can be performed manually, or with the assistance of instruments, such as the ThermoBrite hybridization oven, the VP 2000 Processor, or the XMatrix(TM) processing instrument (all available commercially from Abbott Molecular, Inc.).

Detection of FISH signals from a hybridized and washed sample can be accomplished to allow subsequent analysis in real time, such as by an operator who views fluorescence directly at a microscope, or to allow later analysis, by recording images. Detection generally involves the use of an appropriate source of excitation light for each fluorophore in use in the assay (e.g., light passed through an appropriate filter, or light from a laser of an appropriate wavelength); fluorescently emitted light is then passed through an appropriate filter, and it can be viewed directly by an operator and/or photographed with a camera, such as a film-based or digital camera, which may be connected to a computer.

### 3. Enumeration

The FISH signals of a plurality of nuclei in the sample are enumerated. The plurality of nuclei may comprise at least 50, 75, or 100 nuclei. Enumeration can be achieved, for example, by making a list containing an entry for each examined nucleus and how many of each of the FISH signals it contained, and/or by counting the number of nuclei that had each observed combination of FISH signal quantities. Such a list can contain possible but unobserved combinations of FISH signal quantities as well. The list can be reorganized by making entries for each observed combination of FISH signal quantities, accompanied by their frequency (see, e.g., Table 2 below). The information obtained by enumerating FISH signals is to be interpreted in view of at least one artifactual deletion frequency, discussed below.

### 3. Artifactual deletion frequency

The deletion assay methods comprise providing at least one artifactual deletion frequency. As discussed in Section, F.4 above, a nucleus for any sample can lack FISH signals from one or several target probes for reasons other than a deletion or rearrangement; for example, in the case of a fine needle aspirate biopsy sample, including formalin-fixed, paraffin embedded samples, some nuclei in the sample may be truncated. It is also possible that the target hybridization site(s) in a fraction of the nuclei may not be sufficiently accessible during the hybridization step of the FISH procedure owing to cell compression or other artifact arising from fine needle biopsy technique. In addition, the frequency with which FISH signals are observed can be influenced by the quality of and effects of aging on the tissue preparations being studied. Thus, not every nucleus lacking a FISH signal from the target probe is indicative of an actual genetic deletion.

Therefore, in order to mitigate the potential for false positives (in which a deletion is called erroneously), at least one artifactual deletion frequency is provided in order to distinguish between results from a sample indicative of cells having a deletion of the target of the assay, and results from a sample in which the causes of absent target probe signals are likely other than the underlying genotype, such as the technical sources of error discussed above.

An artifactual deletion frequency can be determined based on a control assay, using samples of cells known not to have a deletion of the target gene or genes. The accuracy of the artifactual deletion frequency determined from control samples may be optimized relative to a test sample by using control samples of cells matched closely to the test sample of cells with respect to the sample preparation method.

For example, non-neoplastic tissue samples of the same tissue type as the precancerous/cancerous/potentially cancerous samples to be tested for loss of a gene or genes of interest can serve as controls. In the case of assays on prostate cancers, non-neoplastic tissue samples can be obtained from patients without cancer who are undergoing surgery solely for benign prostate hyperplasia (BPH) for control purposes. In the case of breast cancers, samples can be obtained from patients undergoing surgery solely for prophylactic or reduction purposes.

As discussed above, especially in Sections B and E.4, the artifactual deletion frequency is also influenced by other biological variables affecting both FISH signals and relative nuclear volume in tumor sections, such as the condensation of the DNA, the size and geometry of the nucleus, the thickness of the sample sections, the assay design (the signal distance in 3 or 4 color FISH assay depends partially on the genomic distance of the probes), and the growth rate and ploidy status (higher likelihood of higher levels of ploidy convey larger volume nuclei). In addition to these nuclear variables, the actual FISH signal itself (the "spot size") may occupy more three-dimensional space in decondensed nuclear chromatin (typical open DNA packing of expressed genes) in comparison to condensed regions of the genome (DNA of repressed genes for example). Moreover, individual FISH signals may be more difficult to interpret if DNA replication has already taken place, since G2 nuclei exhibit "doublet" or paired spot counts due to the duplication of the cell's DNA. These doublets are physically linked paired spots, i.e., touching or linked by a thread, or adjacent (with a gap smaller than the diameter of the largest signal or spot). In some embodiments, scoring criteria can be adjusted to ensure that this effect does not distort the results of the assays (see Varella-Garcia M et al., EGFR fluorescence in situ hybridisation assay: guidelines for application to non-small-cell lung cancer, J Clin Pathol. 2009; 62:970-977). This can include counting physically linked paired spots and/or adjacent spots as one signal only. Spots that are adjacent but separated by at least the diameter of the largest signal can be counted as separate signals. On the other hand, prior to DNA replication each signal shows a single dot-like hybridization signal ("singlet") and these are also counted as one signal only. Several of these variables such as ploidy, nuclear size, and chromatin condensation can vary between normal control samples and tumor specimens. Thus, these factors may be difficult to fully account for in negative controls used to establish an artifactual deletion frequency for interphase FISH assays; this can be of particular importance in samples with low-tumor cell content or additional subclonal genomic changes. Thus, it can be advisable to use a relatively stringent statistical threshold for calling deletions, discussed further below.

In some methods, at least two, or more, artifactual deletion frequencies are provided. For example, artifactual deletion frequencies for hemizygous and homozygous deletions can be provided. Also, separate artifactual deletion frequencies can be provided for deletions that lead to absence of FISH signals from different combinations of probes, for example, deletions that affect only the target probe, deletions that affect the target probe and the centromeric flanking probe closest to the target probe, deletions that affect the target probe and the telomeric flanking probe closest to the target probe, and deletions that affect the target probe, the centromeric flanking probe closest to the target probe, and the telomeric flanking probe closest to the target probe. In methods in which a probe set comprising more than two flanking probes is used, artifactual deletion frequencies can be provided for each probe within a given set so that deletions affecting the flanking probes more distant from the target probe can be considered as well.

An advantage of providing separate artifactual deletion frequencies is that the FISH signals missing due to sources of error, such as truncation events, can be more variable from nucleus to nucleus than the genotype of a clonal population, such that the artifactual deletion frequency for any given type of deletion (e.g., a hemizygous deletion that affects the target probe and the telomeric flanking probe closest to the target probe) is lower than it would be if at least one centromeric flanking probe were not also used in the assay, because some of the sources of error such as truncation events may affect the at least one centromeric flanking probe as well.

Reduction of the artifactual deletion frequency can be very helpful depending on the cell type under analysis, in that some cell types are especially prone to truncation artifacts. For example, in a recent study of truncation losses of signals by interphase FISH in 5-micron histological sections, it was found that about 20% of normal bone marrow nuclei exhibited signal losses due to truncations. In contrast, ~60% of nuclei in normal liver sections exhibited truncation losses (Wilkens L et al. 2005. Standardised fluorescence in situ hybridisation in cytological and histological specimens. Virchows Arch 2005; 447: 586-592). This variation in false positive rates for detecting losses is thought to be due to the cutting artifacts of truncation that have an increasing influence when the much larger, irregularly shaped liver cell nuclei are more frequently truncated during the preparation of 5-micron sections.

### 5. Determining at least one apparent deletion frequency

The information obtained by enumerating FISH signals is analyzed to determine at least one apparent deletion frequency. An apparent frequency is an observation of how often the target probe is missing in a sample. It should be noted that this step may occur with any timing relative to the step of providing at least one artifactual deletion frequency. It is also the case that other rearrangements, such as inversions, result in deletions. Much as with artifactual deletion frequencies, in some embodiments, at least two, or more, apparent deletion frequencies are determined. In the interests of efficiency, it is generally advisable to determine apparent deletion frequencies and provide artifactual deletion frequencies in pairs amenable to comparison (e.g., artifactual and apparent deletion frequencies for a hemizygous deletion that affects the target probe and the telomeric flanking probe closest to the target probe). In any event, in order to perform the next step, at least one apparent deletion frequency must be for the same type of deletion event as at least one artifactual deletion frequency.

### 6. Detection deletions or rearrangements of gene(s) of interest

Whether the sample comprises cells with a deletion or rearrangement (resulting in deletion) is determined based on whether at least one apparent deletion frequency is significantly greater than at least one artifactual deletion frequency. As discussed above, the threshold for significance can be chosen according to the desired level of specificity versus sensitivity using a number of statistical approaches. Thus, multiple artifactual deletion frequencies can be provided, and multiple apparent deletion frequencies can be determined. This step can additionally comprise determining whether multiple types of deletion are present. For example, the presence of two complete sets of FISH signals from the flanking and target probes (2 sets of 3 in the case of a single target gene) indicates that a nucleus does not have a deletion of the target tumor suppressor (schematized in Fig. 2B, left diagram). Absence of one or both of the target probe FISH signals for single targets, indicates an apparent hemizygous (Fig. 2B, center) or homozygous (Fig. 2B, right) deletion, respectively. Information about the size of the apparent deletion can be obtained from whether one or more of the flanking probes are also absent. The examples of Fig. 2B right and center are indicative of relatively small deletions in which only the target probe was affected.

In another example, the presence of two sets of FISH signals from the flanking and target probes (2 sets of 4 in the case of first and second target genes) indicates that a nucleus does not have a deletion of the target tumor suppressor, oncogene, or other target genes. In this example, absence of one or two of the target probe FISH signals indicates an apparent hemizygous or homozygous deletion, respectively. Information about the size of the apparent deletion can be obtained from which of the flanking probes or gene targets are also absent.

Thus, some methods comprise determining whether the sample comprises cells with a deletion chosen from deletions that affect only target probe(s), deletions that affect the one or two target probes and the centromeric flanking probe closest to a first target probe, deletions that affect the one or two target probes and the telomeric flanking probe closest to a second target probe, and deletions that affect both target probes, the centromeric flanking probe closest to the first target probe, and the telomeric flanking probe closest to the second target probe.

In addition to the simple deletion configurations shown in Figure 2B, more complex patterns involving gain or loss of signals may be seen because of additional rearrangements close to the regions containing the flanking control probes. For example, other complex signal configurations bearing additional spots may also arise from complex chromosomal gains due to unbalanced translocations, polysomies, or polyploidy, as in trisomy 21. Any pattern differing from the simple patterns observed in normal nuclei is also usually considered abnormal if it appears in a significant proportion of cells. As with PTEN and other genes, careful evaluation of the number and location of signals in aberrant patterns can provide valuable information of underlying chromosomal change.

### 7. Boundary zones

One or more of the flanking probes used in assays of more than one target gene can have a hybridization site in a boundary zone or distal to the gene(s) of interest relative to a boundary zone. In some embodiments, the at least one first flanking probe hybridizes to a position within or centromeric to a first boundary zone centromeric to one of the genes of interest, and/or the at least one second flanking probe hybridizes to a position within or telomeric to a second boundary zone telomeric to the same or a different gene of interest. In the case of two target genes, one target gene is referred to as the first target gene, and the other is referred to as the second target gene. As for the PTEN example, use of flanking probes with hybridization sites placed in such a way can be helpful because the hybridization site of a flanking probe positioned in this way should be less likely to be deleted than if it were proximal to the gene(s) of interest relative to the boundary zone. Use of such a probe set can raise the frequency of deletions which give patterns as in the center and right of Fig. 2B. These patterns have signals from flanking probes that hybridize centromerically and telomerically to the (presumably partly or completely deleted) gene(s) of interest, and these patterns are less likely to arise repeatedly through truncation artifacts in which a section of the volume of the nucleus is ablated, because it would be necessary for the chromosome to have been arranged in a manner such that a loop of chromatin containing the gene(s) of interest but neither flanking probe hybridization site extended into the ablated nuclear volume. Boundary zones and positions of hybridization sites relative thereto are discussed in greater detail above; that discussion is relevant here as well.

### I. Assays for distinguishably detecting small and large deletions

It is possible that cells in which both copies of at least two genes of interest have been deleted have undergone one or more separate deletion events of different sizes. Described herein are methods of distinguishably detecting deletion events of different sizes.

### 1. Probe sets

In methods pertaining to a more complex arrangement, as exists for the 21 q22-23 chromosomal ∼ 2MB region, which contains no less than thirteen genes according to the present draft of the human genome sequence, probe sets of at least four probes are used, so that a finer level of deletion detection can occur. The probe set comprises at least two target probes that hybridize to the genes of interest (e.g., the HMGN1 gene and the DSCAM gene), at least a first flanking probe that hybridizes to a position centromeric to the gene(s) of interest (e.g., ERG), at least a second flanking probe that hybridizes to a position telomeric to the gene(s) of interest (e.g., TMPRSS2), and optionally at least one of either at least a third flanking probe that hybridizes to a position centromeric to the hybridization site of the first flanking probe (e.g., DYRK1 A), or at least a fourth flanking probe that hybridizes to a position telomeric to the hybridization site of the second flanking probe (e.g., U2AF1). See Amono, K, et al. "Association study between the Down syndrome cell adhesion molecule (DSCAM) gene and bipolar disorder" (2008) Psychiatric Genetics 18 (1) 1-10; Cuddapah,S, et al. "Genomic profiling of HMGN1 Reveals and Association with Chromatin at Regulatory Regions" (2011) Molecular and Cellular Biology 31, 700-709. The probe set may additionally comprise a centromeric or pericentromeric probe.

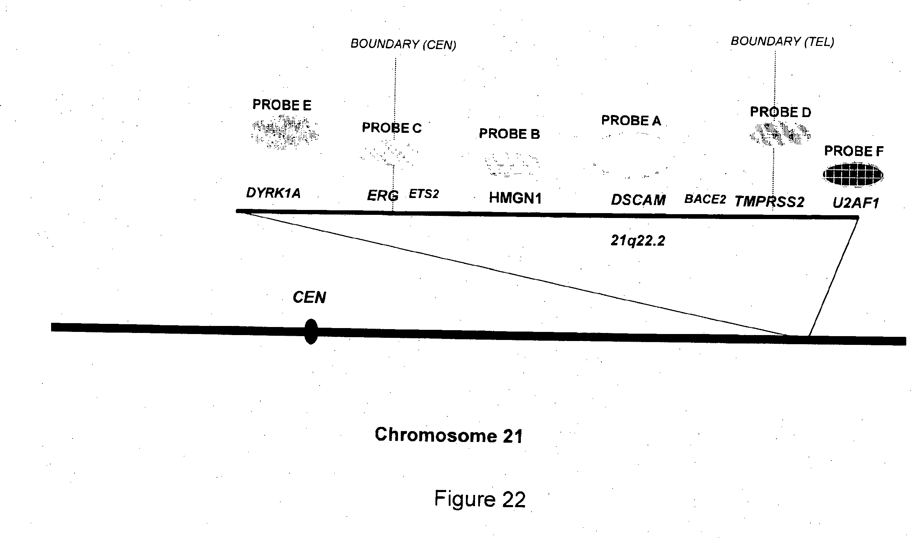

The probe set may comprise two subsets which comprise partially overlapping probes. The two subsets can be used in a two-part assay in which at least one differently positioned flanking probe is used in the second or reflex part of the assay. For example, Figure 22 provides an illustration of one possible configuration of probes described above for a two part assay for small and large DSCAM deletions in which a centromeric probe, a TMPRSS2 probe, a DSCAM probe, and a ERG probe are used in the first part, and a centromeric probe, a DYRK1 A probe, a DSCAM probe, and a U2AF1 probe are used in the second part. If a deletion exceeds the region bounded by the vertical dotted lines in Fig. 22, one or more of the flanking probes should be affected; very large deletions would be indicated when the *TMPRSS2* or *ERG* probes are affected. For example, it is possible for a deletion to result in the loss of the entire region from *DSCAM* to the telomere, which would affect the *DSCAM, TMPRSS2,* and *U2AF1* probes.

### 2. Enumeration; artifactual and apparent deletion frequencies

The steps of enumerating FISH signals, providing artifactual deletion frequencies, and determining apparent deletion frequencies for the above examples are relatively complex because there are multiple probes to enumerate, and artifactual and apparent frequencies for multiple types of deletions can be provided and determined, respectively.

For example, at least one first artifactual deletion frequency can be provided for deletions of *DSCAM* or *HMGN1* with endpoints between the at least first and at least second flanking probes (i.e., the flanking probes closest to the target probes in the centromeric and telomeric directions), and at least one second artifactual deletion frequency can be provided for deletions of the gene(s) of interest with endpoints between the two most distal flanking probes, wherein at least one of the endpoints is not between the at least first and at least second flanking probes. That is, the first artifactual deletion frequency corresponds to deletions encompassing one or both of *DSCAM* or *HMGN1* but not the binding sites of the first and second flanking probes, and the second artifactual deletion frequency corresponds to larger deletions that do encompass a flanking probe binding site.

Alternatively or in addition, at least one first artifactual deletion frequency can be provided for deletions of *DSCAM* or *HMGN1* with endpoints between the at least first and at least second flanking probes in which one of the endpoints is between *DSCAM* and *HMGN1,* and at least one second artifactual deletion frequency can be provided for deletions of the gene(s) of interest in which the endpoints are not between DSCAM and *HMGN1.* That is, the first artifactual deletion frequency corresponds to deletions encompassing one of *DSCAM* or *HMGN1* but not the other, and the second artifactual deletion frequency corresponds to deletions encompassing both (because neither endpoint is between the genes).

The above discussion is applicable regardless of whether the flanking probes are hybridized to the sample(s) together or in a two-part assay.

### 3. Determining whether the sample comprises cells with a small or large deletion

Determination of whether the sample comprises cells with a deletion of the gene(s) of interest that affects the hybridization site of any of the flanking probes used is based on whether the relevant apparent deletion frequency is significantly greater than the relevant artifactual deletion frequency, as described above.

A sample in which the target probe has an apparent hemizygous or homozygous deletion frequency significantly greater than the appropriate artifactual deletion frequency is determined to comprise a small deletion or deletions only (not affecting any flanking probes) if none of the flanking probes have apparent deletion frequencies significantly greater than the appropriate artifactual deletion frequency.

If the target probe and at least one flanking probe have apparent deletion frequencies significantly greater than the appropriate artifactual deletion frequency, then it is likely that a large deletion is present.

It should be noted that the possibility of an unusual pair of small deletions in which, for example, only the hybridization of a flanking probe is deleted on one copy of chromosome 21 and only the hybridization site of the target probe is deleted on the other copy of chromosome 21 can generally be ruled out based on the tendency of FISH signals from the same chromosome to be near one another; thus, in the case of a hemizygous large deletion, the FISH signals from the unaffected chromosome should be near one another.

It is possible for both small and large deletions to be present; in these cases, the target probe has an apparent homozygous deletion frequency significantly greater than the corresponding artifactual homozygous deletion frequency, and at least one flanking probe has an apparent hemizygous deletion frequency significantly greater than the corresponding artifactual hemizygous deletion frequency. In cases in which the large deletion has resulted in the loss of two flanking probe hybridization sites (e.g., a loss of the entire region from a gene of interest to the telomere which affects two flanking probes with hybridization sites telomeric to the gene(s) of interest), then two flanking probes would have an apparent hemizygous deletion frequency significantly greater than the corresponding artifactual hemizygous deletion frequency.

Homozygous deletion of the gene(s) of interest wherein there is at least one large deletion may be indicative of a metastasizing or metastatic tumor; a metastasizing tumor is able to give rise to at least a second tumor at another location in the body, and the second tumor is a metastatic tumor. In some such embodiments, one copy of the gene(s) of interest is missing due to a small deletion and one copy is missing due to a large deletion. In some methods, both copies of the gene(s) of interest are missing due to large deletions. There may be at least one large deletion which affects at least two flanking probe hybridization sites located telomeric to the gene(s) of interest. The more distal (with respect to the gene(s) of interest) of said at least two flanking probe hybridization sites may be at least 5, 7.5, 10, or 12.5 Mb from the gene(s) of interest.

The method for detecting small and large deletions may comprise performing FISH on a first cellular sample comprising a plurality of cells with a first probe subset comprised by the probe set and performing FISH on a second cellular sample from the same individual as the first cellular sample comprising a plurality of cells with a second probe subset comprised by the probe set; the genes of interest are DSCAM and HMGN1; the first probe subset comprises a flanking probe that hybridizes to TMPRSS2 and a flanking probe that hybridizes to ERG; and the second probe subset comprises a flanking probe that hybridizes to DYRK1 A and a flanking probe that hybridizes to U2AF1. When DSCAM and/or HMGN1 deletions are detected in this method, analysis of whether at least one of the hybridization sites of the flanking probes is deleted along with DSCAM and/or HMGN1 can be used to determine whether the deletion is a large deletion.

### 4. Boundary zones

As in the methods of section H, the at least one first flanking probe may hybridize to a position within or centromeric to a first boundary zone centromeric to the gene(s) of interest, and/or the at least one second flanking probe hybridizes to a position within or telomeric to a second boundary zone telomeric to the gene(s) of interest.

It is also possible for the third and/or fourth flanking probes to be positioned within a boundary zone or distal to the gene(s) of interest relative to a boundary zone. That is to say, the at least one third flanking probe may hybridize to a position centromeric to a first distal boundary zone centromeric to the hybridization site of the at least one first flanking probe, and/or the at least one fourth flanking probe hybridizes to a position telomeric to a second distal boundary zone telomeric,to the hybridization site of the at least one second flanking probe.

Boundary zones and positions of hybridization sites relative thereto are discussed in greater detail in sections above; that discussion is relevant here as well.

### J. Probe sets and kits

Probe sets may be prepared according to the methods described above.

Described herein are probe sets comprising at least one probe that hybridizes to *DSCAM* or *HMGN1,* at least one probe that hybridizes to *TMPRSS2* or *U2AF1,* and at least one probe that hybridizes to ERG. In such probe sets, the at least one probe that hybridizes to *DSCAM* or *HMGN1* serves as a target probe, and the at least one probe that hybridizes to *TMPRSS2* or *U2AF1* and the at least one probe that hybridizes to ERG serve as flanking probes. The probe set may comprise at least one additional flanking probe that hybridizes to *DYRK1A*.

The probe set may comprise at least one probe that hybridizes to *DSCAM* derived from at least one of the BACs RP11-139D12, RP11-907P24, RP11-280O17, RP11-183I12, RP11-281F3, RP11-1113M13, and RP11-123A7. The probe set may comprise at least one probe that hybridizes to *HMGN1* derived from at least one of the BACs RP11.-137J13 and RP11-348C15. The probe set may comprise at least one probe that hybridizes to *TMPRSS2* derived from at least one of the BACs RP11-35C4, CTD-3095D11 and RP11-671L10. The probe set may comprise at least one probe that hybridizes to *ERG* derived from at least one of the BACs RP11-476D17 and RP11-951121. The probe set may comprise at least one probe that hybridizes to *DYRK1A* derived from at least one of RP11-105024, RP11-777J19 and CTD-3140L2. The probe set may comprise at least one probe that hybridizes to *U2AF1* derived from at least one of RP11-446L19 and CTD-2601A22. These and all other BACs mentioned herein are available from the Roswell Park Cancer Institute, Buffalo, New York, and the BACPAC Resources Center at Children's Hospital and Research Center at Oakland, California. Sequence information is available, for example, from the NCBI Clone Registry (http://www.ncbi.nlm.nih.gov/projects/genome/clone/).

Below is a table providing information about hybridization site locations on chromosome 21 (coordinates are as in version GRCh37/h19, February 2009) for selected BACs that can be used in probe sets according to the disclosure.

| | Start | End |
|---|---|---|
| ***HMGN1(21q22.2)*** | | |
| RP11-137J13 | 40705310 | 40858474 |
| RP11-348C15 | 40598841 | 40764635 |
| | | |

| ***DSCAM* (21q22.2)** | | |
|---|---|---|
| RP11-139D12 | 41318920 | 41468615 |
| RP11-907P24 | 41464872 | 41657742 |
| RP11-280O17 | 41558372 | 41699925 |
| RP11-183I12 | 41671029 | 41831863 |
| RP11-281F3 | 41782687 | 41975589 |
| RP11-1113M13 | 41860823 | 42000124 |
| RP11-123A7 | 42002863 | 42168411 |
| | | |

| ***ERG (21q22.2)*** | | |
|---|---|---|
| RP11-476D17 | 39682968 | 39863671 |
| RP11-951I21 | 39899621 | 40089103 |
| | | |

| ***TMPHSS2 (21q22.3)*** | | |
|---|---|---|
| RP11-35C4 | 43254164 | 43420344 |
| CTD-3095D11 | 43051013 | 43079214 |
| RP11-671L10 | 42787192 | 42954776 |
| | | |

| ***U2AF1*** | | |
|---|---|---|
| RP11-446L19 | 44237045 | 44550450 |
| CTD-2601A22 | 44456262 | 44606970 |
| | | |

| ***DYRK1A(21q22.13)*** | | |
|---|---|---|
| RP11-105O24 | 38795458 | 38951057 |
| RP11-777J19 | 38719389 | 38887895 |
| CTD-3140L2 | 38881424 | 38942365 |

The probe set may comprise probes that hybridize to DSCAM, HMGN1, TMPRSS2, and ERG, wherein the probe(s) that hybridize(s) to DSCAM is/are derived from the BACs RP11-846G17, RP11-907P24, RP11-280O17, or a combination thereof; and the probe(s) that hybridize(s) to HMGN1 is/are derived from the BACs RP11-8137J13, RP11-348C15, or both; the probe(s) that hybridize(s) to TMPRSS2 is/are derived from the BACs RP11-35C4, CTD-3095D11, or both; and the probe(s) that hybridize(s) to ERG are derived from the BACs RP11-476D17, RP11-951I21, or both.

The probe set may comprise at least one centromeric or pericentromeric probe. This can be used for chromosome enumeration. Examples of pericentromeric probes include probes derived from the BACs CTD-2251 K12, RP11-47B13, CTD-2314J10 or a combination thereof.

The probe set may consist of probes derived from RP11-139D12, at least one of RP11-476D17 and RP11-951I21, and at least one of RP11-35C4 and CTD-3095D11.

### K. BRIEF DESCRIPTION OF THE DRAWINGS

The accompanying drawings, which are incorporated in and constitute a part of this specification, illustrate several embodiments of the invention and together with the description, serve to explain the principles of the invention.
Figure 1. Schematic depiction of large interstitial deletion of part of a chromosome arm. In this example several cytobands are lost after the deletion has taken place.
Figure 2. Schematic depiction of probe set configurations (panel A) and typical interpretations (panel B) using three-color interphase FISH analysis of nuclei. Panel A shows the configuration of the hybridization sites of the probes on a schematic chromosome. Probe A is labeled with a first color, represented as hatching (in fluorescence microscopy, this color could be red, for example); the target probe ("Tum Sup") is labeled with a second color, represented as white (in fluorescence microscopy, this color could be green, for example); and probe B is labeled with a third color, represented as black (in fluorescence microscopy, this color could be blue, for example). In panel B a normal nucleus (left) will have three pairs of each color signal. For simple hemizygous loss (center nucleus) of the Turn Sup gene, a "white" FISH signal is missing (i.e., only one signal is present), but probes A and B flanking the tumor suppressor gene remain present in duplicate. For homozygous loss (right nucleus) both "white" FISH signals are deleted whilst flanking probes are retained.

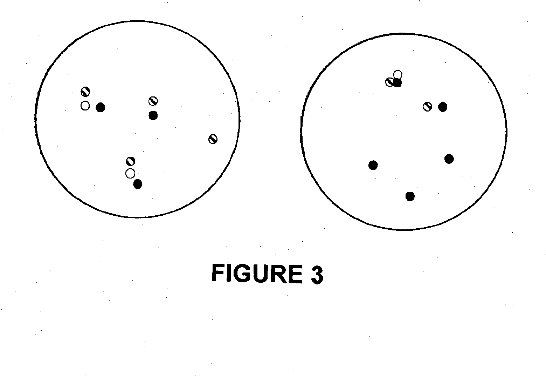

Figure 3. Possible FISH signals from cells having undergone complex rearrangements. The FISH signals in this schematic illustration are generated by the same probe set as in Fig. 2. The pattern at left could be generated by a cell in which two duplications of a region comprising the hybridization site of flanking probe A and one duplication of a region comprising the hybridization site of flanking probe B has occurred. The pattern at right could be generated by a cell in which the tumor suppressor has been hemizygously deleted and three extra copies of a region comprising the hybridization site of probe B have resulted from duplication events.

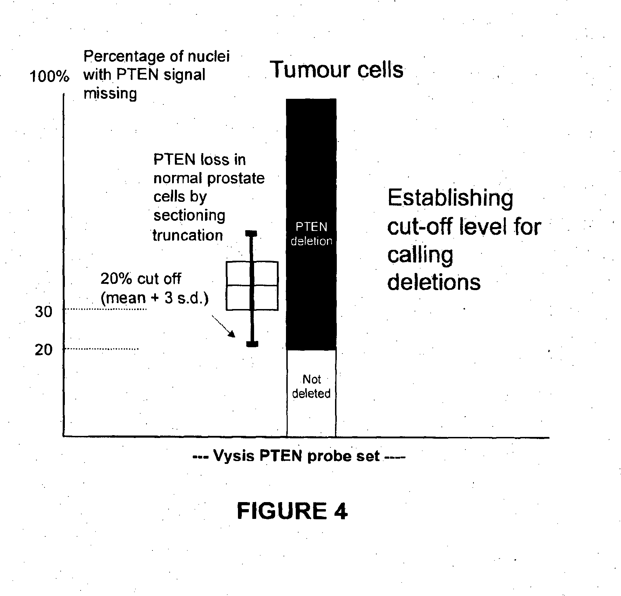

Figure 4. Illustration of thresholds for scoring deletions using control normal prostate cells and *PTEN* deletion detection using a two-color probe set (Vysis Inc.) in which one probe hybridizes to *PTEN* and the other probe is a chromosome enumeration probe for chromosome 10 with a centromeric hybridization site.

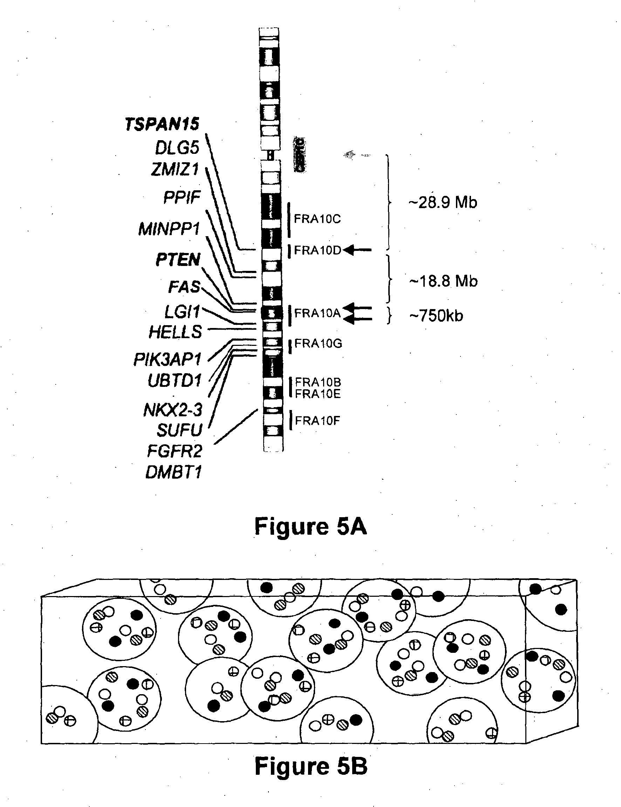

Figure 5. FISH assay design using probes hybridizing to *TSPAN15, PTEN,* and *FAS.* Panel A is an illustration of hybridization sites of probes that can be used to detect *PTEN* deletion events. The three probe set comprises a red *PTEN* probe which is flanked by the violet *TSPAN15* on the centromeric side, and by the green *FAS* gene on the telomeric side. Panel B is a three dimensional schematic depiction of four color interphase FISH using normal cells that will be expected to have two red *(PTEN;* represented as white), green (*FAS*; represented as diagonal hatching), blue (chromosome 10 enumeration probe; cross hatching) and violet (*TSPAN15;* represented as black) spots per nucleus. The blue centromere probe is used to determine if monosomy 10 may be present. Because of the close proximity of the hybridization sites of the flanking probes to that of the target probe, truncations tend to impact the flanking probes and the target probe together; scoring algorithms for deletion can be designed with improved discrimination between real deletion events that do not affect the flanking probes and the target probe and the truncations caused by sectioning. In the assay analyses of normal nuclei such as in the schematic section shown here can be used to provide threshold levels of artifactual deletion frequency for each probe due truncation exclusion during sectioning.

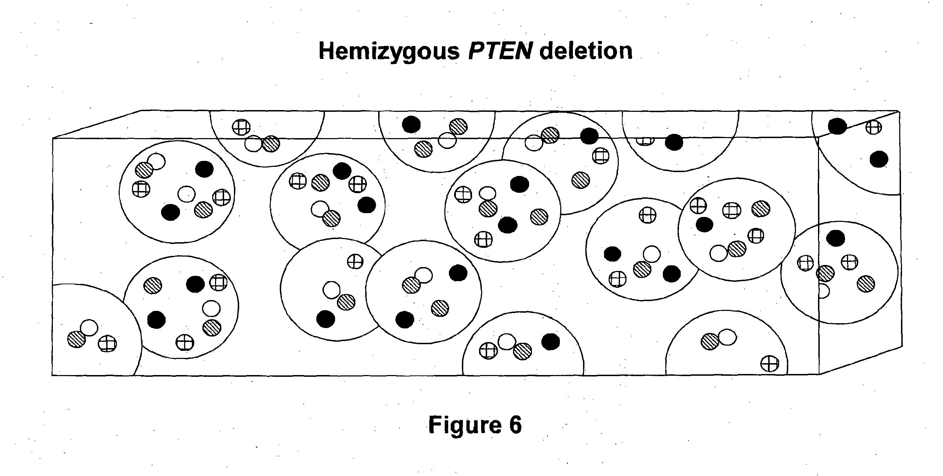

Figure 6. Three dimensional schematic depiction of four color interphase FISH using cells hemizygously deleted for *PTEN* that will be expected to have one red (*PTEN;* represented as white), two green (*FAS*; represented as diagonal hatching), two blue (chromosome 10 enumeration probe; cross hatching) and two violet (*TSPAN15;* represented as black) spots per nucleus.

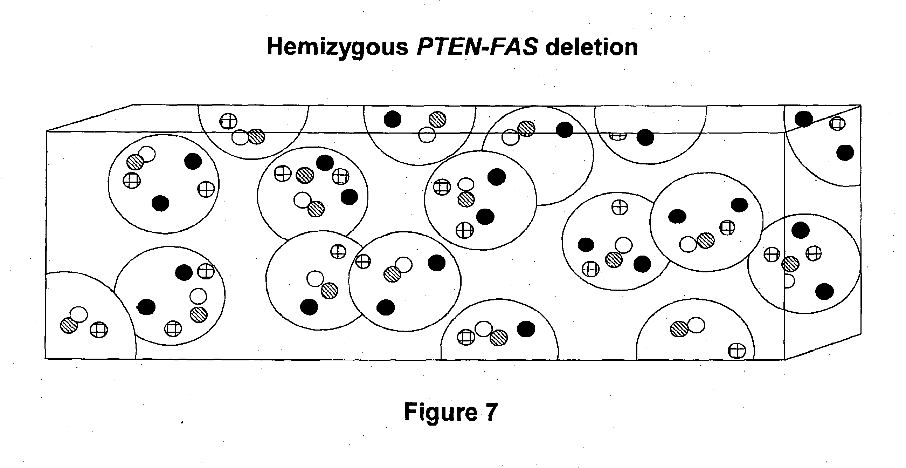

Figure 7. Three dimensional schematic depiction of four color interphase FISH using cells hemizygously deleted for a region comprising *PTEN* and *FAS* that will be expected to have one red (*PTEN;* represented as white), one green (*FAS*; represented as diagonal hatching), two blue (chromosome 10 enumeration probe; cross hatching) and two violet (*TSPAN15;* represented as black) spots per nucleus.

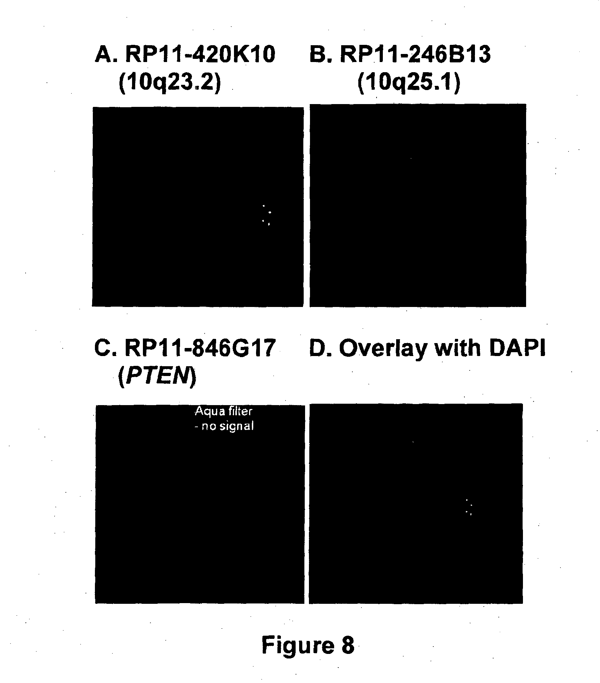

Figure 8. Metaphase FISH with three probes on chromosomes from a cell with a small homozygous *PTEN* deletion. Figure 8A. FISH signals from a centromeric flanking probe derived from RP11-420K10, with a hybridization site at 10q23.2. Two doublet FISH signals were observed for this probe. Figure 8B. FISH signals from a telomeric flanking probe derived from RP11-246B13, with a hybridization site at 10q25.1. Two doublet FISH signals were observed for this probe as well. Figure 8C. Absence of FISH signals from RP11-846G17, which would hybridize to *PTEN* if present. Only some bleedthrough of nonspecific DNA staining from the DAPI channel was visible. Figure 8D. Overlay of the three channels shown in panels A-C and the DAPI channel.

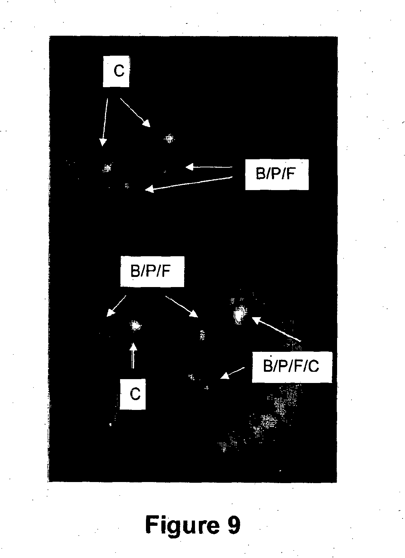

Figure 9. Shown are two nuclei comprising multiple FISH signals from *BMPR1A, PTEN, FAS,* and chromosome 10 centromeric probes. The upper nucleus shows two centromeric signals (C) and two clusters of *BMPR1A*/*PTEN*/*FAS* (B/P/F) signals. The lower nucleus appears to be a post-replication nucleus in which there are two clusters of *BMPR1A*/*PTEN*/*FAS* plus centromeric signals (B/P/F/C), and two B/P/F clusters, one of which is near a centromeric signal. It is possible that two centromeric signals are not resolved, or that one was lost due to a truncation event.

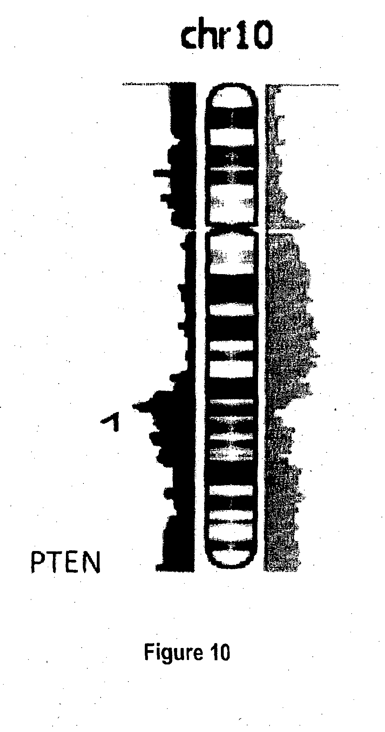

Figure 10. Copy number profile for prostate cancer samples versus normal reference DNA from CGH data. The profile on the left indicates extent of loss in the cancer samples, while the profile on the right indicates extent of gain. The arrowhead indicates a peak in the loss profile corresponding to the position of *PTEN.* See Example 1 for further discussion.

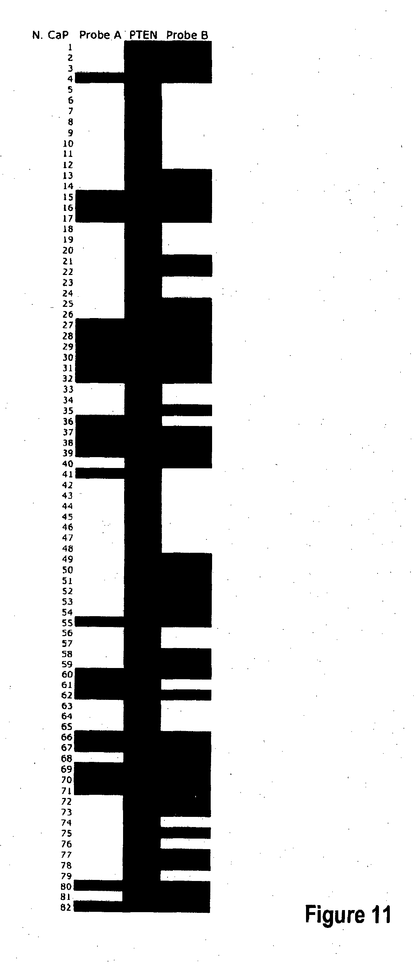

Figure 11. FISH results for 82 prostate cancer samples with a hemizygous *PTEN* deletion. Probes with 1 missing FISH signal are indicated by shading; thus a sample that lost only 1 *PTEN* signal has shading only in the *PTEN* row, while a sample in which both flanking probes were lost as well has shading in all three rows. See Example 3 for further discussion.

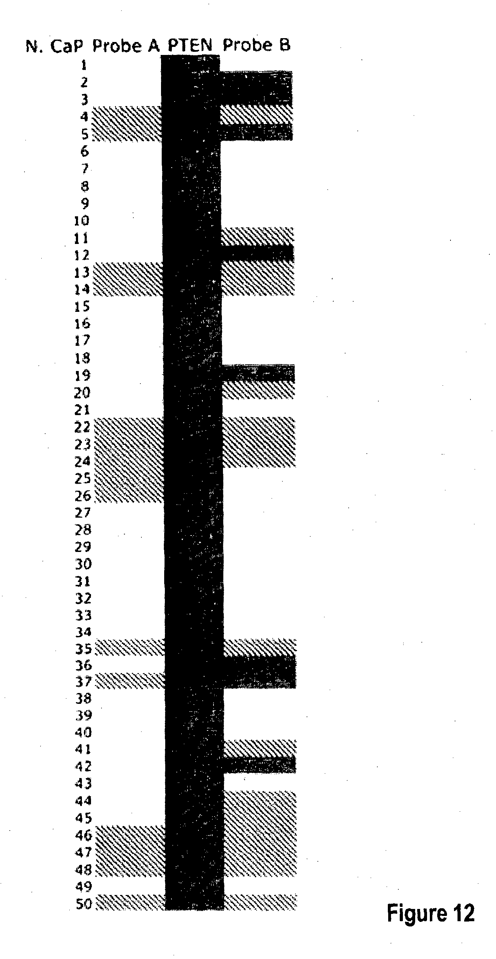

Figure 12. FISH results for 50 prostate cancer samples with a homozygous *PTEN* deletion. Plotting is as in Fig. 11 except that shading indicates homozygous loss and diagonal hatching indicates hemizygous loss. See Example 3 for further discussion.

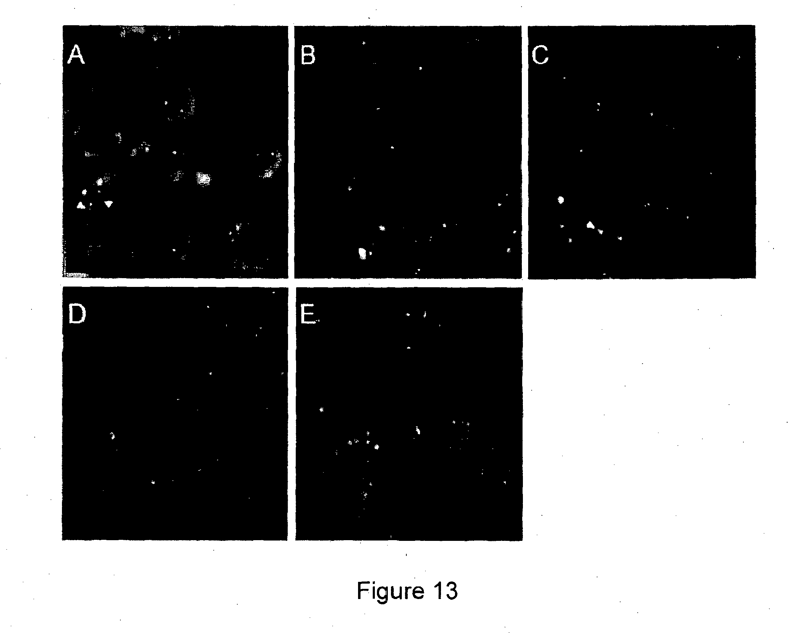

Figure 13. Representative FISH microscopy images for various *PTEN* deletion statuses. White arrowheads indicate *PTEN* FISH signals. In addition to PTEN, probes used were as in Fig. 9. The cells in panel A had 2 of each FISH signal (normal). The cells in panel B frequently lacked one *PTEN* FISH signal (small hemizygous *PTEN* deletion). The cells in panel C frequently lacked one *PTEN* FISH signal and one probe A FISH signal (large hemizygous *PTEN* deletion). The cells in panel D frequently lacked both FISH signals for *PTEN* and probe B (large homozygous deletion). The cells in panel E frequently lacked two *PTEN* FISH signals (small homozygous deletion).

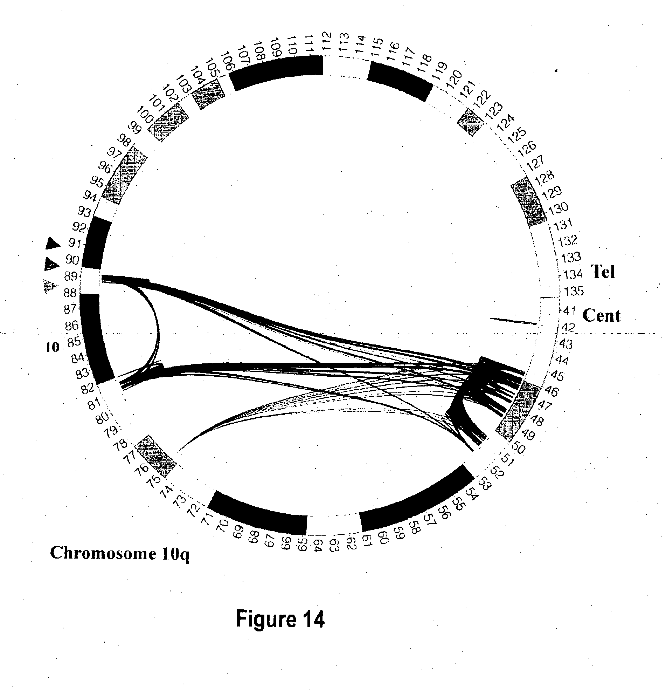

Figure 14. Circular ideogram of chromosome 10q in which segmental duplications are connected by the curved lines on the interior of the circle. The three arrowheads outside the circle indicate, from bottom to top, the hybridization sites of probe A, the *PTEN* probe, and probe B, respectively.

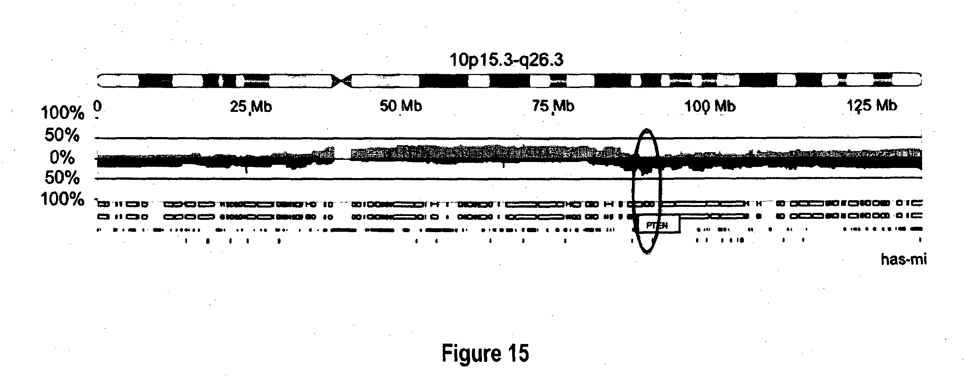

Figure 15. Chromosome 10 is shown with the location of *PTEN* circled. A plot of losses and gains generated from the CGH copy number data of Example 1 is shown below the schematic of the chromosome. In the bottom two rows are CNV and segmental duplication sites.

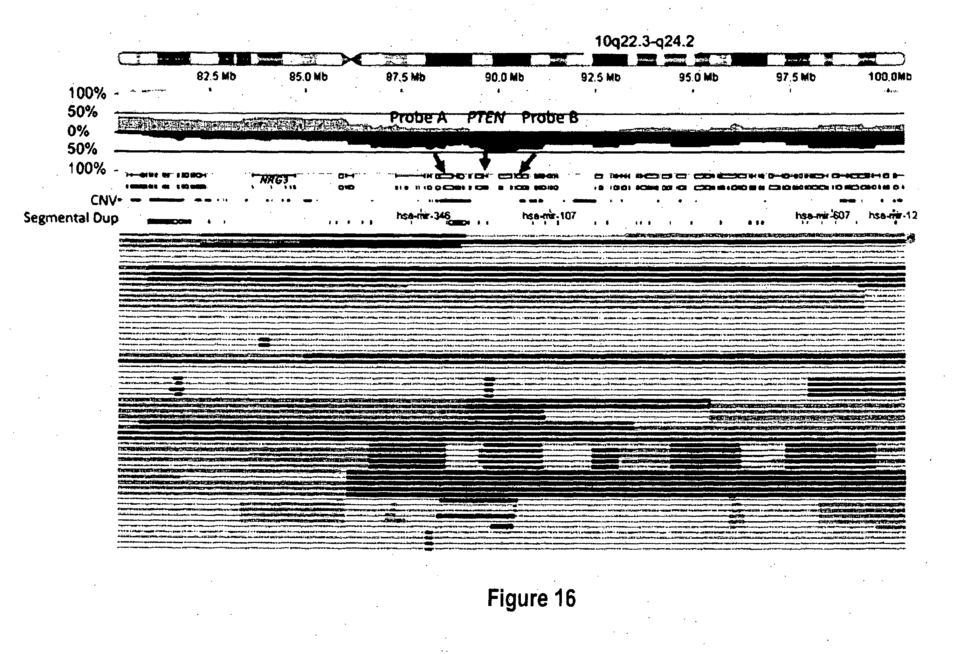

Figure 16. An enlargement of the data from Figure 15 in the neighborhood of *PTEN* is shown. Locations of Probe A and Probe B from Fig. 13 et al. are indicated. CNV and segmental duplications are shown in the 3^{rd} and 5^{th} rows beneath the CGH data. Below that is a representation of deleted areas in individual prostate cancer samples (thick lines indicate deletions).
Figure 17. Probe subsets for a 2-part method for distinguishing small and large *PTEN* deletions. In the first part of the method, FISH can be performed with a flanking probe hybridizing to *TSPAN15,* a target probe hybridizing to *PTEN,* and a flanking probe hybridizing to *FAS.* A centromeric probe can also be used. Boundary zones, where breakpoints of small deletions seem to occur most often, are indicated by dotted lines. The position of a segmental duplication cluster (SD) is also indicated. In the second part ("reflex assay"), the flanking probe hybridize to *BMPR1A* and *SUFU.* Each part of the assay interrogates whether a region relatively close to *PTEN* on one side is affected by a large deletion and provides a more distant probe on the other side that would be affected in even larger deletions; in the case of *SUFU,* losses could be caused by deletions extending from *PTEN* all the way to the telomere.

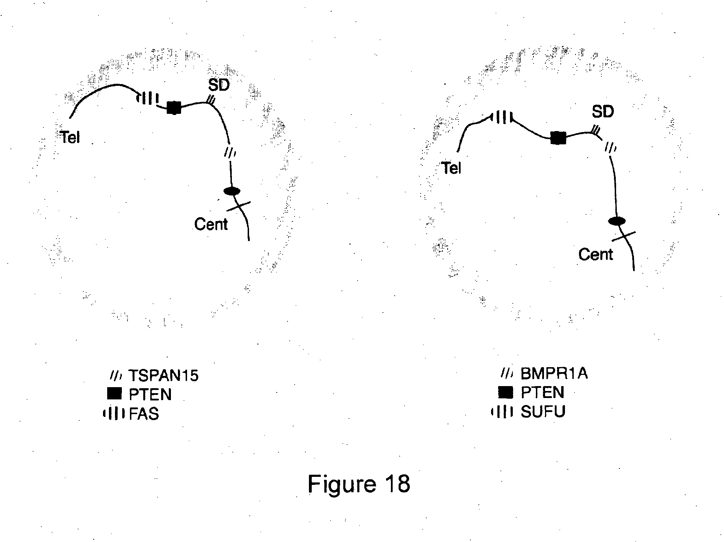

Figure 18. Schematic representation of FISH signals from probes as in Fig. 17 bound to an intact chromosome for the two parts of the assay to distinguish small and large *PTEN* deletions.

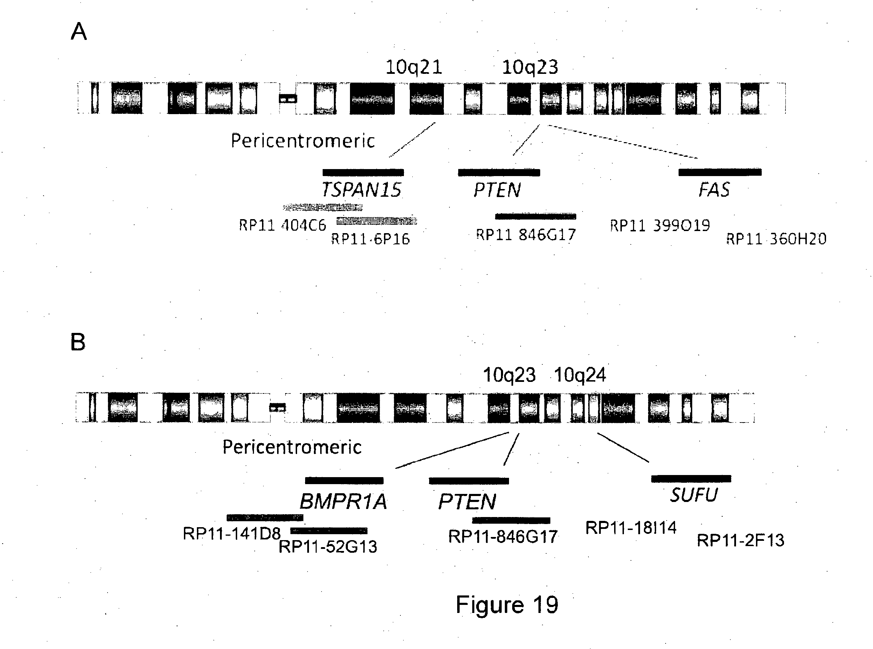

Figure 19. Schematic representation of probe hybridization sites for the probes of the two part assay as in Fig. 17 along chromosome 10 and BACs from which the probes can be derived. Panel A shows the probes of the first subset and Panel B shows the probes of the second subset (for the reflex assay).

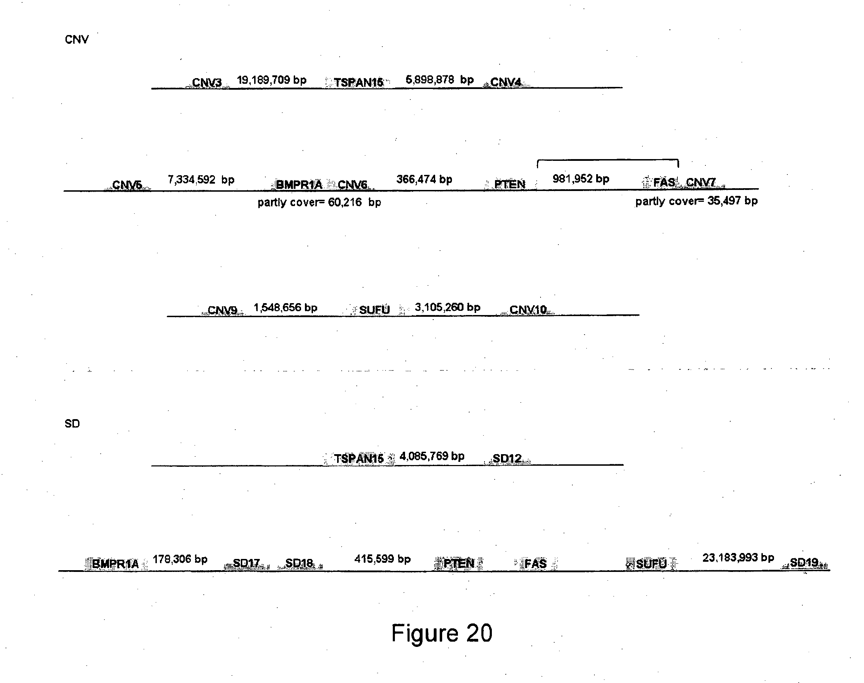

Figure 20. Relative positions of CNV and segmental duplication loci near *PTEN, BMPR1A, SUFU, TSPAN15,* and *FAS.*

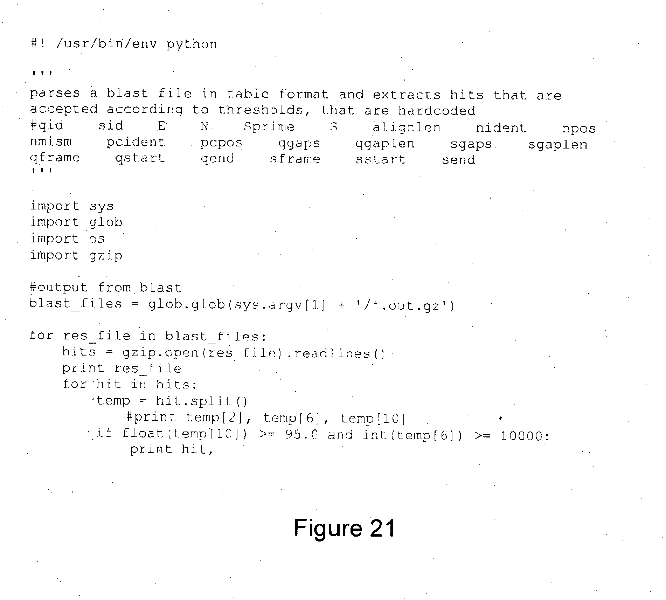

Figure 21. Python scripting language source code for extraction of hits from a blast file with 95% identity and length > 10000 bp.
Figure 22. Schematic depiction of probe set configurations using four-color fish to detect smaller and larger deletions. This shows the hybridization sites of probes on a blown up section of region 21q22.2. Target Probe A shown with vertical lines (DSCAM) is labeled in a 1^{st} color (Aqua, for example). Target Probe B shown with horizontal stripes (HMGN1) is labeled in a second color (Gold, for example). Flanking Probe C shown checkered (ERG) is labeled in a third color (for example, orange). Flanking probe D, shown in solid (5' TMPRSS2) is labeled in a fourth color (for example, green). The diagram also shows centromeric flanking probe E in dots (DYRK1A, labeled in green fluor, for example) and telomeric flanking probe F in crosshatch (U2AF1-labeled in gold fluor, for example) that are used in cases where 3 signals of the first four probes are lost, and a larger deletion boundary is in effect. In a four-color test, if probes A and B are lost, Probes C and D (Orange and Green fluors) would remain. If Probes A, B, and C are lost, Probe D (Green fluor) remains, and a follow-up 3-color test with any of the further flanking Probes can be used to determine the deletion boundary.

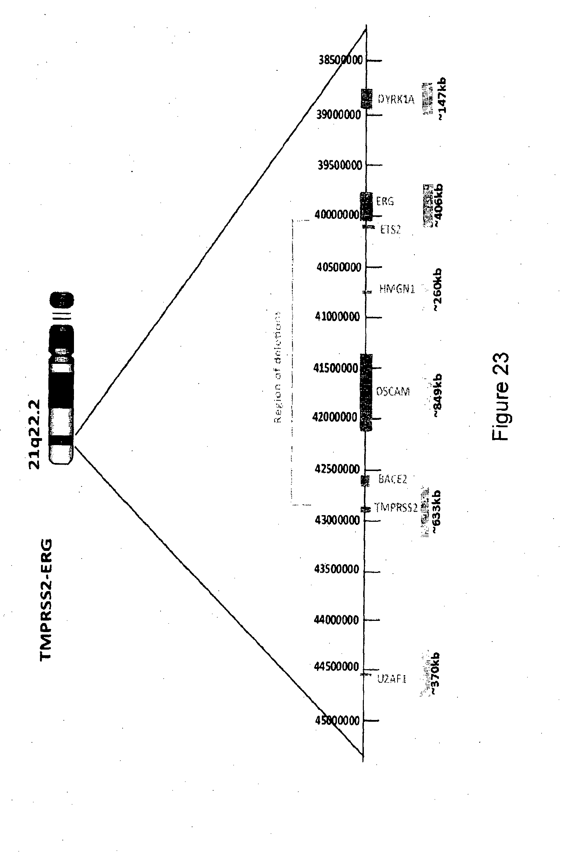

Figure 23. Map of the 21q22.13-21q22.3 region showing gene and exemplary probe locations and an indication of a zone within which deletions may occur.

### EXAMPLES

Reference will now be made in detail to embodiments of the invention, aspects and results of which are illustrated in the accompanying drawings.

### Example 1. Analysis of copy number variation, segmental duplication, and comparative genomic hybridization data for chromosome 10; probe site selection

CGH data from Liu et al., Nat. Med. 2009; 15:559-65 were analyzed in silico. *In silico* copy number analysis of the chromosome 10q region in 58 metastatic CaP samples from 14 patients (Liu W et al. Copy number analysis indicates monoclonal origin of lethal metastatic prostate cancer, Nat. Med. 2009; 15:559-65) was performed applying rank segmentation, with a significance threshold of 1.0×10⁻⁶ and a minimum of 5 probes per segment (Nexus Copy Number v.4; BioDiscovery, EI Segundo, CA). Genomic imbalances were assigned as either gain [log(3/2) or threshold of 0.2] or loss [log(1/2) or threshold of -0.3], each determined by two Copy Number Transitions (CNTs), as defined by Ferreira BI et al., Array CGH and gene-expression profiling reveals distinct genomic instability patterns associated with DNA repair and cell-cycle checkpoint pathways in Ewing's sarcoma, Oncogene 2008; 27:2084-2090.

Data showing areas of loss near *PTEN* are shown in Fig. 16. The average abundance of loci in the population of metastatic CaP samples relative to the reference was also determined and plotted (Fig. 15 and 16; see also Fig. 10). It was determined that the abundance declined by more than 20% in the region from 81.5 Mb to 89.67 Mb (i.e., the ∼8 Mb centromeric to *PTEN),* representing a copy number transition in the population.

Copy Number Variation (CNV) data was obtained from the Sanger Institute's CNV Project (http://www.sanger.ac.uk/humgen/cnv/ in ASCII text format. The data available corresponded to 269 distinct samples collected by the international consortium HapMap (http://hapmap.ncbi.nlm.nih.gov/). The downloaded files were then filtered and only regions pertaining to chromosome 10 were transferred to a spreadsheet. Overlapping regions were selected manually on Microsoft Excel. Segmental duplication data was obtained by employing Blast (Altschul SF et al., J. Mol. Biol. 1990; 215:403-410) alignments using the assembled chromosome 10 as the reference database.

Individual segmental duplications obtained from the Segmental Duplication Database (She X et al., Shotgun sequence assembly and recent segmental duplications within the human genome, Nature 2004; 431:927-930, http://humanparalogy.gs.washington.edu/) were then aligned to the chromosome in automatic fashion and the resulting hits tabulated for each segment in the database. Tables for the more than 9000 sequences in Segmental Duplication Database were filtered, and only hits (alignments) with more than 95% homology and longer than 10 kilobases were automatically selected by computer script (Figure 21). Contiguous regions were then selected manually in the same fashion as the CNV segments. The cut off value considered to group contiguous alignments (hits) was 50 kilobases of distance. Another cutoff value of 100 kilobases of distance was employed in order to group the resulting clusters.

It was determined that chromosome 10 contained clusters of segmental duplications (SDs) and CNVs in the regions listed in Table 1.

**Table 1. Chromosome 10 Segmental duplications and CNVs**

| High density region | Start (bp) | End (bp) | Length (bp) |
|---|---|---|---|
| CNV1 | 42,004,899 | 42,760,575 | 755,676 |
| CNV2 | 45,341,719 | 49,121,538 | 3,779,819 |
| CNV3 | 50,641,980 | 51,595,172 | 953,192 |
| CNV4 | 76,904,943 | 77,440,455 | 535,512 |
| CNV5 | 80,945,468 | 81,722,592 | 777,124 |
| CNV6 | 88,505,038 | 89,299,742 | 794,704 |
| CNV7 | 90,825,044 | 91,007,466 | 182,422 |
| CNV8 | 98,779,953 | 98,952,462 | 172,509 |
| CNV9 | 102,141,840 | 102,555,726 | 413,886 |
| CNV10 | 107,519,652 | 107,743,529 | 223,877 |
| CNV11 | 110,448,977 | 110,633,486 | 184,509 |
| CNV12 | 122,625,702 | 122,891,863 | 266,161 |
| CNV13 | 124,253,065 | 124,444,805 | 191,740 |
| CNV14 | 125,047,207 | 125,259,149 | 211,942 |
| CNV15 | 127,443,890 | 127,776,692 | 332,802 |
| CNV16 | 134,076,220 | 135,240,498 | 1,164,278 |

| High density region | Start (bp) | End (bp) | Length (bp) |
|---|---|---|---|
| SD1 | 41,991,393 | 42,173,845 | 182,452 |
| SD2 | 42,531,265 | 42,689,258 | 157,993 |
| SD3 | 45,492,329 | 46,843,228 | 1,350,899 |
| SD4 | 47,023,808 | 47,533,536 | 509,728 |
| SD5 | 47,731,989 | 47,900,982 | 168,993 |
| SD6 | 48,376,799 | 48,697,638 | 320,839 |
| SD7 | 48,865,543 | 49,055,736 | 190,193 |
| SD8 | 50,735,042 | 51,157,549 | 422,507 |
| SD9 | 51,275,627 | 51,628,828 | 353,201 |
| SD10 | 52,104,752 | 52,214,149 | 109,397 |
| SD11 | 57,043,647 | 57,055,423 | 11,776 |
| SD12 | 75,091,834 | 75,143,840 | 52,006 |
| SD13 | 80,936,173 | 80,980,415 | 44,242 |
| SD14 | 81,081,817 | 81,275,000 | 193,183 |
| SD15 | 81,379,701 | 81,625,517 | 245,816 |
| SD16 | 81,959,635 | 82,002,788 | 43,153 |
| SD17 | 88,743,560 | 88,770,949 | 27,389 |
| SD18 | 88,890,157 | 89,250,617 | 360,460 |
| SD19 | 127,598,385 | 127,609,227 | 10,842 |
| SD20 | 135,233,079 | 135,363,669 | 130,590 |

The coordinates in Table 1 refer to positions in chromosome 10 of UCSC version NCBI36/hg18 (Mar. 2006) of the human genome.

Based on the locations of the segmental duplications, CNV, and population copy number transition, the TSPAN15 and BMPR1A loci were selected as hybridization sites for centromeric flanking probes. The FAS and SUFU loci were selected as hybridization sites for telomeric flanking probes.

### Example 2. Three-color FISH with a sample having a homozygous PTEN deletion.

Metaphase chromosomes from cells of the PC3 cell line (Beheshti B et al., Evidence of chromosomal instability in prostate cancer determined by spectral karyotyping (SKY) and interphase fish analysis, Neoplasia 2001; 3:62-9) were fixed and hybridized with three distinguishably labeled probes prepared using the RP11-420K10 BAC, with a hybridization site at 10q23.2 (labeled green); the RP11-246B13 BAC, with a hybridization site at 10q25.1 (labeled red); and RP11-846G17, with a hybridization site at *PTEN* (labeled aqua). The chromosomes were also counterstained with DAPI (blue). Images in the red, green, aqua, and blue channels were obtained by fluorescence microscopy. Images from a representative set of chromosomes are shown in Figure 8, in which four FISH signals were visible in the green and red channels (panels A and B). There were 4 FISH signals since the diploid genome has been replicated but not yet segregated at metaphase. No aqua FISH signals were observed for the *PTEN* probe (panel C) although slight DAPI fluorescence was visible in this channel. Panel D shows an overlay of the three images of panels A-C and the DAPI fluorescence from the blue channel.

### Example 3. Deletion mapping by four-color interphase FISH

Four color interphase FISH was performed on 132 samples of cancerous prostate tissue deleted for at least one copy of *PTEN.* The 132 samples were a subset of 330 cancerous prostate clinical tissue samples taken at McGill University and the University of Toronto. Details about these samples appear in the tables below.

**BREAKDOWN OF THE TOTAL 330 PATIENTS**

| | |
|---|---|
| Radical Prostatectomies | 134 (41%) |
| Hormone refractory/Metastatic tumors | 196 (59%) |
| Total | 330 |

**BREAKDOWN OF THE 132 SAMPLES WITH A HEMI- OR HOMOZYGOUS PTEN DELETION**

| | |
|---|---|
| Radical Prostatectomies | 86 |
| Hormone refractory/Metastatic tumors | 46 |
| Total | 132 |

The probes used were a probe derived from the BACs RP11-141D8 and RP11-52G13 ("probe A"; hybridization site centromeric to *PTEN*); a *PTEN* probe derived from the BAC RP11-846G17; and a probe derived from the BACs RP11-399019 and RP11-360H20 ("probe B"; hybridization site telomeric to *PTEN*). The probes were labeled with distinguishable fluorophores by nick translation. Positional information for the BAC clones can be obtained from the Human March 2006 assembly of the UCSC Genome Browser 1. Also used was the chromosome 10 centromeric probe SpectrumAqua labeled CEP10, Vysis Abbott Molecular, Des Plaines, IL, USA.

Analysis of the 132 samples indicated that 82 of them had hemizygous *PTEN* deletions and 50 had homozygous *PTEN* deletions, based on presence of 1 or 0 *PTEN* probe FISH signals in at least 30% of the cells. Additionally, the presence or absence of probes A and B was enumerated to determine whether the deletion(s) encompassed the hybridization sites of these probes as well. Results are listed in Table 2 below and shown in Figs. 11-13.

**Table 2. Extents of deletions**

| Hemizygous *PTEN* deletions | | Homozygous *PTEN* deletions | |
|---|---|---|---|
| Probes Affected | Frequency | Probes Affected | Frequency |
| *PTEN* | 37% | *PTEN (0)* | 48% |
| *PTEN* and Probe B | 32% | Probe A (1), *PTEN* (0), Probe B (1) | 22% |
| Probe A and *PTEN* | 3% | *PTEN* (0) and Probe B (0) | 12% |
| Probe A, *PTEN,* and Probe B | 28% | *PTEN* (0) and Probe B (1) | 10% |
| | | Probe A (1), *PTEN* (0), Probe B (0) | 4% |
| | | Probe A (1) and *PTEN* (0) | 4% |

In the column for homozygous *PTEN* deletions, the parenthesized numbers indicate how many FISH signals from a given probe were present; thus, for example, "Probe A (1), *PTEN* (0), Probe B (1)" indicates that either one chromosome was deleted for *PTEN* only and the other chromosome was deleted for the whole region from Probe A to Probe B, or one chromosome was deleted for *PTEN* and Probe B, and the other chromosome was deleted for Probe A and *PTEN.*

The minimum size of deletions affecting only *PTEN* was estimated as 176 kb. The size range of the largest deletions, affecting Probe A, *PTEN,* and Probe B, was estimated as at least 2.5 Mb.

### Example 4. Resolution of PTEN status in samples difficult to interpret by two color FISH

A set of 91 formalin-fixed paraffin embedded samples from radical prostatectomies with unknown clinical outcome at the time of study were analyzed using both two-color interphase FISH with the commercially available *PTEN* and centromeric probes from Abbott Inc., and by four color FISH, using a probe set in which probe A was prepared from the BACs RP11-141 D8 and RP11-52G13 and the *PTEN* probe and probe B were as in Example 3.. Six samples were identified in which results differed between the two assays. These results are listed in Table 3 below.

The analysis criteria for the two color assay were: at least 70% of nuclei with 2 *PTEN* signals and two chromosome 10 centromeric probes: no copy change. 25%-30% of nuclei with simultaneous loss of one *PTEN* signal missing but presence of two centromeric probes: inconclusive. Greater than 30% of nuclei with one *PTEN* signal missing and both centromeric probes retained: hemizygous deletion. 30%-100% of nuclei with two *PTEN* signals missing and centromeric probes retained: homozygous deletion.

The analysis criteria for the four color assay were established after truncation artifacts were established for each probe in a given set. They were: at least 80% of nuclei with 2 *PTEN* signals and retaining flanking probes: no copy change. 18%-20% of nuclei with one *PTEN* signal missing whilst retaining both flanking probes: inconclusive. Greater than 20% with one *PTEN* signal missing but simultaneously retaining both flanking probes: hemizygous deletion. 20%-100% of nuclei with two *PTEN* signals missing which may be accompanied by simultaneous losses of one, both or neither flanking probes: homozygous deletion.

These criteria were based on a requirement that, to call a deletion, the apparent frequency must have been greater than the artifactual deletion frequency plus three standard deviations; these values were 30% for the two color assay and 20% for the four color assay; see the tables below showing control results for the two color and four color probe sets. Artifactual deletion frequencies were measured using control samples from noncancerous prostate samples from biopsies and/or radical prostatectomies performed on patients with benign prostate hyperplasia. Use of three standard deviations to set a significance threshold in FISH assays is discussed in Ventura et al., J. Mol. Diagn. 2006; 8:141-151.

**Table 3. Control results - artifactual deletion frequency with 4-color probe set**

| **Prostate samples** | **2 CEP10**/ **2 *BMPR1A*/ *1 PTEN*/ 2 *FAS*** |
|---|---|
| control 1 | 12 |
| control 2 | 5 |
| control 3 | 4 |
| control 4 | 10 |
| control 5 | 12 |
| control 6 | 8 |
| control 7 | 2 |
| control 8 | 8 |
| control 9 | 4 |
| control 10 | 13 |
| **Average** | **7.8** |
| **St dev** | **3.9** |
| **3 st dev** | **11.7** |
| **Average+3 st dev** | **19.5** |

**Table 4. Control results - artifactual deletion frequency with 2-color probe set**

| **Prostate samples** | **2 CEP10/1 PTEN (Vysis)** |
|---|---|
| control 1 | 18 |
| control 2 | 17 |
| control 3 | 14 |
| control 4 | 20 |
| control 5 | 15 |
| control 6 | 10 |
| control 7 | 5 |
| control 8 | 16 |
| control 9 | 18 |
| control 10 | 6 |
| **Average** | **13.9** |
| **St dev** | **5.2** |
| **3 st dev** | **15.6** |
| **Average+3 st dev** | **29.5** |

**Table 5. Detection of PTEN deletion using two and four color PTEN probe sets**

| Sample | Two color assay | Four color assay with Ex. 3 probes |
|---|---|---|
| CaP-1 | No copy change | Hemizygous del |
| CaP-2 | Hemizygous del | No copy change |
| CaP-3 | No copy change | Hemizygous del |
| CaP-4 | No copy change | Homozygous del |
| CaP-5 | Hemizygous del | No copy change |
| CaP-6 | Hemizygous del | No copy change |
| CaP-7 | Hemizygous del | No copy change |
| CaP-8 | Hemizygous del | No copy change |
| CaP-9 | Inconclusive | Hemi- and homozygous del |
| CaP-10 | Inconclusive | Hemizygous del |
| CaP-11 | Inconclusive | Hemizygous del |
| CaP-12 | Inconclusive | No copy change |
| CaP-13 | Inconclusive | No copy change |
| CaP-14 | Inconclusive | No copy change |
| CaP-15 | Inconclusive | No copy change |
| CaP-16 | Inconclusive | No copy change |
| CaP-17 | Inconclusive | No copy change |
| CaP-18 | Homozygous del | No copy change |
| CaP-19 | Inconclusive | Hemizygous del |
| CaP-20 | Hemizygous del | No copy change |

"Hemi- and homozygous del" indicates that significant numbers of cells were present with both types of deletion, consistent with an initial deletion of one copy of *PTEN,* followed by clonal expansion, with a second deletion event resulting in a homozygously deleted subpopulation.

Thus, the four color assay was able to resolve samples 9-17 and 19 that were inconclusive according to the two color assay. Additionally, several samples appear to have given a false positive result in the two color assay (presumably due to an above-average number of truncation effects), and samples 1, 3, and 4 appear to have given a false negative result. Finally, in sample 9, the two-color assay apparently did not detect the hemizygously deleted population of cells.

It is thought that the improved specificity and sensitivity of the four-probe assay results from the lower artifactual deletion frequency resulting from having the flanking probes A and B positioned closer to the target of the assay (*PTEN*) but still at locations where many deletions should not affect the FISH signals from the flanking probes.

### Example 5. Boundary zone identification and FISH probe preparation for p16.

CGH data comparing average genomic copy number from melanoma cell samples to reference cells is obtained from [http://www.broadinstitute.org/tumorscape/pages/portalHome.jsf], described in [Beroukhim R et al., The landscape of somatic copy-number alteration across human cancers, Nature 2010; 463:899-905]. The nearest copy number transition zone in which the average relative copy number of loci in the population of 111 melanoma samples declines by at least 20% over an interval of at most 15 Mb is identified on the centromeric side of the p16 gene (also known as CDKN2A) at 9p21.

Genome annotations from the Wellcome Trust Sanger Institute [http://www.sanger.ac.uk/humgen/cnv/] for copy number variation polymorphic loci (CNVs) [Casci T. Genome evolution: CNV evolution revisited, Nature Reviews Genetics 2008; 9:814-815] and from the Department of Genome Sciences, University of Washington [http://humanparatogy.gs.washington.edu/] for segmental duplications [Rudd MK et al., Segmental duplications mediate novel, clinically relevant chromosome rearrangements, Hum. Mol. Genet. 2009; 18:2957-62] in the area of the copy number transition zone and the surrounding vicinity are obtained.

At least one CNV in the copy number transition zone or within the 5 Mb centromeric to it is identified. The annotated endpoints of the nearest of the at least one CNV to p16 are referred to below as the distal and proximal CNV endpoints (with respect to proximity to p16).

At least one cluster containing at least four annotated segmental duplications within a 1 Mb range of annotated segmental duplications in the copy number transition zone or within the 5 Mb centromeric to it is identified. The endpoints of the segmental duplication cluster nearest to p16 are defined either by the annotated endpoints of a high density segmental duplication region in the Segmental Duplication Database (She X et al., Shotgun sequence assembly and recent segmental duplications within the human genome, Nature 2004; 431:927-930, http://humanparalogy.gs.washington.edu/) or by the location of the two segmental duplication loci within the 1 Mb range most proximal and distal to p16.

The region bounded by (1) the more distal to p16 of the distal endpoints of the CNV and the segmental duplication cluster and (2) the more proximal to p16 of the proximal endpoints of the CNV and the segmental duplication cluster is identified as a boundary zone.

A centromeric flanking probe is prepared which has a hybridization site whose center is within the 1 Mb extending in the centromeric direction from the edge of the boundary zone distal to p16.

On the telomeric side of p16, at least one CNV and/or at least one segmental duplication cluster is identified within 2 Mb of the telomeric end of the p16 locus. A telomeric flanking probe is prepared which has a hybridization site whose center is within the 1 Mb extending in the telomeric direction from the telomeric end of either the CNV or the segmental duplication cluster.

A target probe is prepared which has a hybridization site whose center is within the p16 locus or within 100 kb of either end of the p16 locus.

A FISH probe set comprising the centromeric flanking probe, the telomeric flanking probe, and the target probe can be used to assay for deletions of p16 via interphase FISH. The assays have high accuracy with formalin fixed paraffin embedded samples because truncation artifacts affecting the FISH signals of centromeric flanking probe, the telomeric flanking probe, and the target probe are readily distinguished from cells affected by genetic deletions having at least one endpoint between the telomeric and centromeric flanking probes.

### Example 6. Deletion Mapping of 21q22.13-21q22.3 region by four-color interphase FISH.

Four-color interphase FISH is performed on (FFPE-FNA) samples of prostate tissue:
1. Normal cells
2. hemizygous *PTEN* deletions, and
3. homozygous *PTEN* deletions
using the methods described above.

The 21q22.13-21q22.3 (specifically, 21q22.2) region comprises several genes, including *TMPRSS2, HMGN1, DSCAM,* and *ERG1.* The probes to be used are derived from BACs RP11-476D17 (Probe A: centromeric to *HMGN1*); a *HMGN1* probe derived from BACs RP11-137J13 and RP11-348C15; a *DSCAM* probe derived from BACs RP11-139D12, RP11-907P24, RP11-280O17, RP11-183112, RP11-281F3, RP11-1113M13, and RP11-123A7 and a probe derived from RP11-35C4 (probe B; telomeric to *DSCAM*)*.* The probes are labeled distinguishably (e.g., with red, green, aqua, and gold fluorophores) by nick translation. Positional information for the BACs can be obtained from Human (February 2009) assemblies of the UCSC Genome Browser. A chromosome 21 centromeric or pericentric probe may also be used.

Analysis of the samples indicates that a subset of cells having hemizygous PTEN deletion also have a HMGN1 deletion or a DSCAM deletion, or both, as shown by the presence of hybridization signals from Probe A and Probe B but not one or both of the HMGN1 and DSCAM probes. Another subset of cells showing homozygous PTEN deletions shows additional or single deletions. Normal cells show the presence of all the genes.

## Claims

1. A method of conducting a fluorescence in situ hybridization-based assay for deletion of a tumor suppressor gene comprising:
(a) performing fluorescence in situ hybridization (FISH) with a probe set on a cellular sample comprising a plurality of cells,
wherein the probe set comprises at least one first flanking probe that hybridizes to a position centromeric to the tumor suppressor gene, at least one second flanking probe that hybridizes to a position telomeric to the tumor suppressor gene, and at least one target probe that hybridizes to the tumor suppressor gene;
(b) enumerating FISH signals from the at least one first and at least one second flanking probes and the at least one target probe in the plurality of cells;
(c) providing at least one artifactual deletion frequency from a control sample for (1) a deletion that affects only the target probe, (2) a deletion that affects the target probe and the centromeric flanking probe closest to the target probe, (3) a deletion that affects the target probe and the telomeric flanking probe closest to the target probe, or (4) a deletion that affects the target probe, the centromeric flanking probe closest to the target probe, and the telomeric flanking probe closest to the target probe,
the artifactual deletion frequency being chosen from (i) an artifactual hemizygous deletion frequency and (ii) an artifactual homozygous deletion frequency;
(d) determining at least one apparent deletion frequency from the enumerated FISH signals of step (b), for the same type of deletion event as at least one artifactual deletion frequency of step (c),
the apparent deletion frequency being chosen from (i) an apparent hemizygous deletion frequency and (ii) an apparent homozygous deletion frequency,
wherein the at least one apparent deletion frequency comprises an apparent hemizygous deletion frequency if an artifactual homozygous deletion frequency was not provided in step (c), and wherein the at least one apparent deletion frequency comprises an apparent homozygous deletion frequency if an artifactual hemizygous deletion frequency was not provided in step (c); and
(e) determining whether the sample comprises cells with a hemizygous deletion of the tumor suppressor gene based on whether the apparent hemizygous deletion frequency is significantly greater than the artifactual hemizygous deletion frequency, or determining whether the sample comprises cells with a homozygous deletion of the tumor suppressor gene based on whether the apparent homozygous deletion frequency is significantly greater than the artifactual homozygous deletion frequency.

2. The method of claim 1, wherein the probe set further comprises at least one third flanking probe that hybridizes to a position centromeric to the hybridization site of the first flanking probe and at least one fourth flanking probe that hybridizes to a position telomeric to the hybridization site of the second flanking probe;
and wherein the method further comprises:
(i) enumerating FISH signals from the at least one third and at least one fourth flanking probes in the plurality of cells;
(ii) providing at least one first artifactual deletion frequency for deletions of the tumor suppressor gene with endpoints between the at least one first and at least one second flanking probes;
(iii) providing at least one second artifactual deletion frequency for deletions of the tumor suppressor gene wherein at least one of the endpoints is not between the at least first and at least second flanking probes;
(iv) determining, from the enumerated FISH signals of step (i), at least one first apparent deletion frequency for deletions of the tumor suppressor gene with endpoints between the at least one first and at least one second flanking probes;
(v) determining, from the enumerated FISH signals of step (i), at least one second apparent deletion frequency for deletions of the tumor suppressor gene wherein at least one of the endpoints is not between the at least one first and at least one second flanking probes; and
(vi) determining whether the sample comprises cells with a small deletion of the tumor suppressor gene based on whether the at least one first apparent deletion frequency is significantly greater than the at least one first artifactual deletion frequency, and determining whether the sample comprises cells with a large deletion of the tumor suppressor gene based on whether the at least one second apparent deletion frequency is significantly greater than the at least one second artifactual deletion frequency.

3. The method of claim 1 or 2, wherein (i) the tumor suppressor gene is *PTEN,* p16, RB1, p53, a tumor suppressor gene located on a human chromosome at a chromosome band chosen from 10q23, 17p13, 13q14, 9q24, and 9p21, or a tumor suppressor gene located on a human chromosome arm chosen from 10q, 17p, 13q, 9p, 1p, 5q, 19q, 20q, 8p, 12p, and 16q, or wherein (ii) the tumor suppressor gene is *PTEN* and at least one first flanking probe comprises a probe that hybridizes to *TSPAN15, BMPR1A,* or *WAPAL* and the at least one second flanking probe comprises a probe that hybridizes to *FAS.*

4. The method of any one of claims 1, 2, or 3, wherein the apparent frequency is significantly greater than the artifactual frequency if p is less than or equal to 0.05 according to a t-test, or the apparent frequency is significantly greater than the artifactual frequency if the apparent frequency exceeds the artifactual frequency by three standard deviations.

5. The method of any one of claims 1-4, wherein the at least one first flanking probe hybridizes to a position within or centromeric to a boundary zone centromeric to the tumor suppressor gene, or the at least one second flanking probe hybridizes to a position within or telomeric to a boundary zone telomeric to the tumor suppressor gene.

6. The method of any one of claims 1-5, wherein the hybridization sites of the at least one target probe and the at least first and second flanking probes have sizes ranging from 50 to 200 kb, or the hybridization site of the at least one target probe is separated from the hybridization sites of the at least first and second flanking probes by a distance ranging from 500 kb to 20 Mb.

7. The method of any one of claims 1-6, wherein the cellular sample is fixed and preserved, or is a formalin-fixed, paraffin-embedded sample with a thickness ranging from 3 to 6 µm.

8. The method of any one of claims 1-6, wherein the cellular sample is prepared by fixation with ethanol or methanol:acetic acid combined with cytocentrifugation, thin layer deposition, a smear, or pipetting onto a microscope slide.

9. A probe set suitable for the method of any of claims 1-8 comprising at least one probe that hybridizes to *PTEN,* at least one probe that hybridizes to *FAS* or *SUFU,* and at least one probe that hybridizes to *WAPAL,* wherein the WAPAL-hybridizing probe is derived from RP11-661D10 wherein each probe is labelled with a different color.

10. A kit for detecting a deletion of a tumor suppressor gene comprising the probe set of claim 9.

11. The probe set of claim 9 for use in detecting a deletion of a tumor suppressor gene in a cellular sample.

## Patentansprüche

1. Verfahren zur Durchführung eines Fluoreszenz-in-situ-Hybridisierungs-Assays zur Deletion eines Tumorsuppressorgens, umfassend:
(a) Durchführen einer Fluoreszenz-in-situ-Hybridisierung (FISH) mit einer Sondenanordnung an einer Zellprobe, die eine Mehrzahl von Zellen umfasst;
wobei die Sondenanordnung wenigstens eine erste flankierende Sonde umfasst, die an eine zu dem Tumorsuppressorgen zentromerische Position hybridisiert, wenigstens eine zweite flankierende Sonde, die an eine zu dem Tumorsuppressorgen telomerische Position hybridisiert, und wenigstens eine Zielsonde, die an das Tumorsuppressorgen hybridisiert;
(b) Spezifizieren der FISH-Signale von den wenigstens einen ersten und der wenigstens einen zweiten flankierenden Sonden und der wenigstens einen Zielsonde in der Mehrzahl von Zellen;
(c) Bereitstellen wenigstens einer artifiziellen Deletionsfrequenz von einer Kontrollprobe für (1) eine Deletion, die nur die Zielsonde betrifft, (2) eine Deletion, welche die Zielsonde und die der Zielsonde am nächsten liegende zentromerische flankierende Sonde betrifft, (3) eine Deletion, welche die Zielsonde und die der Zielsonde am nächsten liegende telomerische flankierende Sonde betrifft, oder (4) eine Deletion, welche die Zielsonde, die der Zielsonde am nächsten liegende zentromerische flankierende Sonde und die der Zielsonde am nächsten liegende telomerische flankierende Sonde betrifft,
wobei die artifizielle Deletionsfrequenz ausgewählt ist aus (i) einer artifiziellen hemizygoten Deletionsfrequenz und (ii) einer artifiziellen homozygoten Deletionsfrequenz;
(d) Bestimmen wenigstens einer scheinbaren Deletionsfrequenz aus den spezifizierten FISH-Signalen aus Schritt (b) für den gleichen Deletionsereignistyp wie wenigstens eine artifizielle Deletionsfrequenz aus Schritt (c),
wobei die scheinbare Deletionsfrequenz ausgewählt ist aus (i) einer scheinbaren artifiziellen hemizygoten Deletionsfrequenz und (ii) einer scheinbaren artifiziellen homozygoten Deletionsfrequenz,
wobei die wenigstens eine scheinbare Deletionsfrequenz eine scheinbare hemizygote Deletionsfrequenz umfasst, wenn in Schritt (c) keine artifizielle homozygote Deletionsfrequenz bereitgestellt worden ist, und wobei die wenigstens eine scheinbare Deletionsfrequenz eine scheinbare homozygote Deletionsfrequenz umfasst, wenn in Schritt (c) keine artifizielle hemizygote Deletionsfrequenz bereitgestellt worden ist; und (e) Bestimmen, ob die Probe Zellen mit einer hemizygoten Deletion des Tumorsuppressorgens umfasst, darauf basierend, ob die scheinbare hemizygote Deletionsfrequenz signifikant höher ist als die artifizielle hemizygote Deletionsfrequenz, oder Bestimmen, ob die Probe Zellen mit einer homozygoten Deletion des Tumorsuppressorgens umfasst, darauf basierend, ob die scheinbare homozygote Deletionsfrequenz signifikant höher ist als die artifizielle homozygote Deletionsfrequenz.

2. Verfahren nach Anspruch 1, wobei die Sondenanordnung ferner wenigstens eine dritte flankierende Sonde umfasst, die an eine zu der Hybridisierungsstelle der ersten flankierenden Sonde zentromerische Position hybridisiert, und wenigstens eine vierte flankierende Sonde, die an eine zu der Hybridisierungsstelle der zweiten flankierenden Sonde telomerische Position hybridisiert;
und wobei das Verfahren ferner folgendes umfasst:
(i) Spezifizieren der FISH-Signale von wenigstens einer der dritten und wenigstens einer der vierten flankierenden Sonden in der Mehrzahl von Zellen;
(ii) Bereitstellen wenigstens einer ersten artifiziellen Deletionsfrequenz für Deletionen des Tumorsuppressorgens mit Endpunkten zwischen der wenigstens einen der ersten und der wenigstens einen der zweiten flankierenden Sonden;
(iii) Bereitstellen wenigstens einer zweiten artifiziellen Deletionsfrequenz für Deletionen des Tumorsuppressorgens, wobei wenigstens einer der Endpunkte nicht zwischen der wenigstens einen der ersten und der wenigstens einen der zweiten flankierenden Sonden liegt;
(iv) Bestimmen aus den spezifizierten FISH-Signalen aus Schritt (i) wenigstens einer ersten scheinbaren Deletionsfrequenz für Deletionen des Tumorsuppressorgens mit Endpunkten zwischen der wenigstens einen der ersten und der wenigstens einen der zweiten flankierenden Sonden;
(v) Bestimmen aus den spezifizierten FISH-Signalen aus Schritt (i) wenigstens einer zweiten scheinbaren Deletionsfrequenz für Deletionen des Tumorsuppressorgens wobei wenigstens einer der Endpunkte nicht zwischen der wenigstens einen der ersten und der wenigstens einen der zweiten flankierenden Sonden liegt; und
(vi) Bestimmen, ob die Probe Zellen mit einer kleinen Deletion des Tumorsuppressorgens umfasst, darauf basierend, ob die wenigstens eine erste scheinbare Deletionsfrequenz signifikant höher ist als die wenigstens eine erste artifizielle Deletionsfrequenz, und Bestimmen, ob die Probe Zellen mit einer großen Deletion des Tumorsuppressorgens umfasst, darauf basierend, ob die wenigstens eine zweite scheinbare Deletionsfrequenz signifikant höher ist als die wenigstens eine zweite artifizielle Deletionsfrequenz.

3. Verfahren nach Anspruch 1 oder 2, wobei (i) das Tumorsuppressorgen *PTEN,* p16, RB1, p53 ist, ein an einem menschlichen Chromosom angeordnetes Tumorsuppressorgen an einem Chromosomenband, ausgewählt aus 10q23, 17p13, 13q14, 9q24 und 9p21, oder ein an einem menschlichen Chromosomenarm angeordnetes Tumorsuppressorgen, ausgewählt aus 10q, 17p, 13q, 9p, 1 p, 5q, 19q, 20q, 8p, 12p und 16q, oder wobei (ii) das Tumorsuppressorgen *PTEN* ist, und wobei wenigstens eine erste flankierende Sonde eine Sonde umfasst, die an *TSPAN15, BMPR1A* oder *WAPAL* hybridisiert, und wobei die wenigstens eine zweite flankierende Sonde eine Sonde umfasst, die an *FAS* hybridisiert.

4. Verfahren nach einem der Ansprüche 1, 2 oder 3, wobei die scheinbare Frequenz signifikant höher ist als die artifizielle Frequenz, wenn p gemäß einem t-Test kleiner oder gleich 0,05 ist, oder wobei die scheinbare Frequenz signifikant höher ist als die artifizielle Frequenz, wenn die scheinbare Frequenz die artifizielle Frequenz um drei Standardabweichungen überschreitet.

5. Verfahren nach einem der Ansprüche 1 bis 4, wobei die wenigstens eine erste flankierende Sonde an eine Position innerhalb oder zentromerisch zu einer Grenzzone, zentromerisch zu dem Tumorsuppressorgen, hybridisiert, oder wobei die wenigstens eine zweite flankierende Sonde an eine Position innerhalb oder telomerisch zu einer Grenzzone, telomerisch zu dem Tumorsuppressorgen, hybridisiert.

6. Verfahren nach einem der Ansprüche 1 bis 5, wobei die Hybridisierungsstellen der wenigstens einen Zielsonde und der wenigstens einen ersten und zweiten flankierenden Sonden Größen zwischen 50 und 200 kb aufweisen, oder wobei die Hybridisierungsstelle der wenigstens einen Zielsonde getrennt ist von den Hybridisierungsstellen der wenigstens einen ersten und zweiten flankierenden Sonden um einen Abstand von 500 kb bis 20 Mb.

7. Verfahren nach einem der Ansprüche 1 bis 6, wobei die Zellprobe fixiert und konserviert ist, oder wobei es eine formalinfixierte, in Paraffin eingebettete Probe mit einer Dicke zwischen 3 und 6 µm ist.

8. Verfahren nach einem der Ansprüche 1 bis 6, wobei die Zellprobe erzeugt wird durch Fixierung mit Ethanol oder Methanol:Ethansäure vereint durch Zytozentrifugation, Dünschichtabscheidung, einen Abstrich, oder Pipettieren auf einen Objektträger.

9. Sondenanordnung, die geeignet ist für das Verfahren nach einem der Ansprüche 1 bis 8, umfassend wenigstens eine Sonde, die an *PTEN* hybridisiert, wenigstens eine Sonde, die an *FAS* oder *SUFU* hybridisiert, und wenigstens eine Sonde, die an *WAPAL* hybridisiert, wobei die an *WAPAL* hybridisierende Sonde von RP11-661D10 stammt, wobei jede Sonde mit einer anderen Farbe markiert ist.

10. Kit zum Nachweisen einer Deletion eines Tumorsuppressorgens, umfassend die Sondenanordnung aus Anspruch 9.

11. Sondenanordnung nach Anspruch 9 zur Verwendung zum Nachweisen einer Deletion eines Tumorsuppressorgens in einer Zellprobe.

## Revendications

1. Procédé permettant d'effectuer un test basé sur l'hybridation in situ en fluorescence pour la suppression d'un gène suppresseur de tumeurs consistant à :
(a) effectuer l'hybridation in situ en fluorescence (FISH) avec un jeu de sondes sur un échantillon cellulaire comprenant une pluralité de cellules,
dans lequel le jeu de sondes comprend au moins une première sonde flanquante qui s'hybride à une position centromérique au gène suppresseur de tumeurs, au moins une seconde sonde flanquante qui s'hybride à une position télomérique au gène suppresseur de tumeurs, et au moins une sonde cible qui s'hybride au gène suppresseur de tumeurs ;
(b) énumérer les signaux FISH a partir d'au moins l'un premier et au moins une seconde sondes flanquantes et l'au moins une sonde cible dans la pluralité de cellules ;
(c) fournir au moins une fréquence de suppression artéfactuelle à partir d'un échantillon de contrôle pour (1) une suppression qui affecte seulement la sonde cible, (2) une suppression qui affecte la sonde cible et la sonde flanquante centrométrique la plus proche de la sonde cible, (3) une suppression qui affecte la sonde cible et la sonde flanquante télomérique la plus proche de la sonde cible, ou (4) une suppression qui affecte la sonde cible, la sonde flanquante centrométrique la plus proche de la sonde cible, et la sonde flanquante télomérique la plus proche de la sonde cible,
la fréquence de suppression artéfactuelle étant choisie parmi (i) une fréquence de suppression hémizygote artéfactuelle et (ii) une fréquence de suppression homozygote artéfactuelle ;
(d) déterminer au moins une fréquence de suppression apparente à partir des signaux FISH énumérés de l'étape (b), pour le même type d'événement de suppression qu'au moins une fréquence de suppression artéfactuelle de l'étape (c),
la fréquence de suppression apparente étant choisie parmi (i) une fréquence de suppression hémizygote et (ii) une fréquence de suppression homozygote,
dans lequel l'au moins une fréquence de suppression apparente comprend une fréquence de suppression hémizygote apparente si une fréquence de suppression homozygote artéfactuelle n'a pas été fournie lors de l'étape (c), et dans lequel l'au moins une fréquence de suppression apparente comprend une fréquence de suppression homozygote apparente si une fréquence de suppression hémizygote artéfactuelle n'a pas été fournie lors de l'étape (c) ; et
(e) déterminer si l'échantillon comprend des cellules avec une suppression hémizygote du gène suppresseur de tumeurs si la fréquence de suppression hémizygote artéfactuelle est significativement plus élevée que la fréquence de suppression hémizygote artéfactuelle, ou déterminer si l'échantillon comprend des cellules avec une suppression homozygote du gène suppresseur de tumeurs si la fréquence de suppression homozygote artéfactuelle est significativement plus élevée que la fréquence de suppression homozygote artéfactuelle.

2. Procédé selon la revendication 1, dans lequel le jeu de sondes comprend en outre au moins un tiers de sonde flanquante qui s'hybride à une position centromérique au site d'hybridation de la première sonde flanquante et au moins un quart de sonde flanquante qui s'hybride à une position télomérique au site d'hybridation de la seconde sonde flanquante ;
et dans lequel le procédé comprend en outre les étapes consistant à:
(i) énumérer les signaux FISH à partir d'au moins un tiers et au moins un quart des sondes flanquantes dans la pluralité de cellules ;
(ii) fournir au moins une première fréquence de suppression artéfactuelle pour des suppressions du gène suppresseur de tumeurs avec des extrémités entre l'au moins une première et au moins une seconde sondes flanquantes ;
(iii) fournir au moins une seconde fréquence de suppression artéfactuelle pour des suppressions du gène suppresseur de tumeurs dans laquelle au moins une des extrémités n'est pas entre l'au moins première et au moins seconde sondes flanquantes ;
(iv) déterminer, à partir des signaux FISH énumérés de l'étape (i), au moins une première fréquence de suppression apparente pour des suppressions du gène suppresseur de tumeurs avec des extrémités entre l'au moins une première et au moins une seconde sondes flanquantes ;
(v) déterminer, à partir des signaux FISH énumérés de l'étape (i), au moins une seconde fréquence de suppression apparente pour des suppressions du gène suppresseur de tumeurs dans laquelle au moins une des extrémités n'est pas entre l'au moins une première et au moins seconde sondes flanquantes ; et
(vi) déterminer si l'échantillon comprend des cellules avec une petite suppression du gène suppresseur de tumeurs si l'au moins une première fréquence de suppression apparente est significativement plus élevée que l'au moins une première fréquence de suppression artéfactuelle, et déterminer si l'échantillon comprend des cellules avec une large suppression du gène suppresseur de tumeurs si l'au moins une seconde fréquence de suppression apparente est significativement plus élevée que l'au moins une seconde fréquence de suppression artéfactuelle

3. Procédé selon l'une des revendications 1 ou 2, dans lequel (i) le gène suppresseur de tumeurs est PTEN, p16, RB1, p53, un gène suppresseur de tumeurs situé sur un chromosome humain à une bande de chromosome choisi parmi 10q23, 17p13, 13q14, 9q24 et 9p21, ou un gène suppresseur de tumeurs situé sur un chromosome humain à un bras de chromosome choisi parmi 10q, 17p, 13q, 9p, 1 p, 5q, 19q, 20q, 8p, 12p et 16q, ou dans lequel (ii) le gène suppresseur de tumeurs est PTEN et au moins une première sonde flanquante comprend une sonde qui s'hybride à TSPAN15, BMPR1A, ou WAPAL et l'au moins une seconde sonde flanquante comprend une sonde qui s'hybride à FAS.

4. Procédé selon l'une quelconque des revendications 1, 2 ou 3, dans lequel la fréquence apparente est significativement plus élevée que la fréquence artéfactuelle si p est inférieur ou égal à 0,05 selon un test t, ou la fréquence apparente est significativement plus élevée que la fréquence artéfactuelle si la fréquence apparente dépasse la fréquence artéfactuelle de trois écarts types.

5. Procédé selon l'une quelconque des revendications 1 à 4, dans lequel l'au moins une première sonde flanquante s'hybride à une position au sein de ou centromérique à une zone frontière centromérique à une position au sein de ou télomérique à une zone frontière télomérique au gène suppresseur de tumeurs.

6. Procédé selon l'une quelconque des revendications 1 à 5, dans lequel les sites d'hybridation de l'au moins une sonde cible et l'au moins les première et seconde sondes flanquantes possèdent des tailles comprises dans la gamme allant de 50 à 200 kb, ou le site d'hybridation de l'au moins une sonde cible est séparé des sites d'hybridation de l'au moins les première et seconde sondes flanquantes par une distance comprise dans la gamme allant de 500 kb à 20 Mb.

7. Procédé selon l'une quelconque des revendications 1 à 6, dans lequel l'échantillon cellulaire est fixé et préservé, ou est un échantillon incorporé à la paraffine fixé sur la formaline avec une épaisseur comprise dans la gamme allant de 3 à 6 µm.

8. Procédé selon l'une quelconque des revendications 1 à 6, dans lequel l'échantillon cellulaire est préparé par fixation avec de l'éthanol ou du méthanol : acide acétique combiné avec une centrifugation, une déposition sur couche mince, un frottis ou un pipetage sur une lame de microscope.

9. Jeu de sondes adapté pour le procédé selon l'une quelconque des revendications 1 à 8 comprenant au moins une sonde qui s'hybride à PTEN, au moins une sonde qui s'hybride à FAS ou SUFU, et au moins une sonde qui s'hybride à WAPAL, dans lequel la sonde s'hybridant à WAPAL est dérivée de RP11-661 D10, dans lequel chaque sonde est marquée avec une couleur différente.

10. Kit de détection pour la suppression d'un gène suppresseur de tumeurs comprenant le jeu de sondes suivant la revendication 9.

11. Jeu de sondes selon la revendication 9 pour une utilisation consistant à détecter la suppression d'un gène suppresseur de tumeurs dans un échantillon cellulaire.
